(19) Europäisches Patentamt
European Patent Office
Office européen des brevets

(11) EP 0 503 537 B1

(12) **EUROPEAN PATENT SPECIFICATION**

(45) Date of publication and mention of the grant of the patent: **14.05.1997 Bulletin 1997/20**

(51) Int. Cl.[6]: **C07D 211/08**, A61K 31/435, C07D 401/12

(21) Application number: **92104004.4**

(22) Date of filing: **09.03.1992**

(54) **6,9 Bis(substituted-amino)benzo-[g]isoquinoline-5,10-diones**

6,9-Bis(substituiert-amino)benzo[9]isoquinoline-5,10-Dione

6,9-bis(amino substitué)benzo[9]isoquinoleines-5,10-diones

(84) Designated Contracting States:
**AT BE CH DE DK ES FR GB GR IT LI LU NL PT SE**

(30) Priority: **08.03.1991 US 665954**
**29.01.1992 US 827302**

(43) Date of publication of application:
**16.09.1992 Bulletin 1992/38**

(73) Proprietor: **THE UNIVERSITY OF VERMONT**
**Burlington, Vermont 05405-0160 (US)**

(72) Inventor: **Krapcho, Paul. A**
**Shelburne, Vermont 05482 (US)**

(74) Representative: **Weber, Manfred, Dr. et al**
**c/o Boehringer Mannheim GmbH,**
**Patentabteilung,**
**Sandhoferstrasse 116**
**68298 Mannheim (DE)**

(56) References cited:
**GB-A- 2 000 029** **US-A- 4 197 249**

- **EUROPEAN JOURNAL OF MEDICINAL CHEMISTRY, vol. 23, no. 1, January 1988, pages 101-106, Paris, FR; M. CROISY-DELCEY et al.: "Aminoalkylamino derivatives of dihydroxy-benzo(g)isoquinoline dione and of trihydroxy-naphto(2,3- g)isoquinoline dione: synthesis and anti-tumor evaluation"**
- **JOURNAL OF MEDICINAL CHEMISTRY, vol. 28, no. 8, August 1985, pages 1124-1126, Washington DC, US; A.P. KRAPCHO et al.: "Syntesis and antineoplastic evaluations of 5.8-bis[(aminoalkyl)amino]-1-azaathracene-9,10-diones".**

Remarks:

- Consolidated with 92908816.9/0575526 (European application no./publication no.) by decision dated 28.08.95.
- The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 503 537 B1

## Description

## BACKGROUND OF THE INVENTION

### Field of the invention

This invention is directed to 6,9 bis(substituted-amino)benzo[g]isoquinoline-5,10-diones, and more particularly, to 6,9-substituents which are (aminoalkyl)amino substituents. These compounds have been shown to have antitumor activity in vitro and in vivo.

### Background

Certain 1,4-bis[(aminoalkyl)amino]anthracene-9,10-diones have been reported which show antitumor activity in clinical trials. Of particular interest has been ametantrone, 1,4-bis{[2-(2-hydroxyethylamino)ethyl]amino}anthracene-9,10-dione and mitoxantrone, 5,8-dihydroxy-1,4-bis{[2-(2-hydroxyethylamino)ethyl]amino}anthracene-9,10-dione. (Zee-Cheng et al., J. Med. Chem., (1978), 21, 291-4; Cheng et al., "Progress in Medicinal Chemistry", Ellis, G.P. and West, G.B., eds.; Elsevier: Amsterdam, 1983; pp. 20, 83 and references cited therein). Mitoxantrone is a broad spectrum oncolytic agent, whose activity is similar to that of the anthracycline antibiotic doxorubicin. Clinical trials have demonstrated that mitoxantrone has particularly promising activity in the treatment of advanced breast cancer, acute leukemia and lymphoma (Legha, Drugs of Today, (1984), 20, 629). Although animal studies have demonstrated a diminished cardiotoxicity in comparison to doxorubicin, some clinical cardiotoxicity has been observed also with mitoxantrone, mostly in patients previously treated with doxorubicin (R. Stuart Harris et al., Lancet, (1984), 219, and references cited therein).

Ametantrone has been reported to be, in animals, about 10-fold less potent and cardiotoxic than mitoxantrone. Because a delayed toxicity is observed only with mitoxantrone after administration of the two drugs by the i.p. route to non-tumor bearing rats at equieffective antitumor dosages, it is suggested that the presence of the 5,8-dihydroxy substitution in mitoxantrone might be implicated in the delayed deaths (Corbett et al., Cancer Chemother. Pharmacol., (1981), 6, 161).

In addition, both mitoxantrone and ametantrone have a remarkable myelodepressive toxicity and both compounds show cross-resistance to cell histotypes developing resistance against doxorubicin mediated by overexpression of glycoprotein P. Such a resistance, which is named multidrug resistance, involves a number of antitumor antibiotics, among which amsacrine and podophyllotoxinic derivatives, and it is one of the main reasons for therapeutical failures in the treatment of solid tumors with said antibiotics.

Therefore, search is required for novel anthracenedione antitumor agents, having a higher therapeutical index than mitoxantrone and being effective both in inhibiting or delaying the growth of those solid tumors which are more refractory to chemotherapeutic treatment (such as lung, breast and colon tumors) and against tumor histotypes developing multidrug resistance.

In the search for safer, active analogues of anthracenediones, compounds bearing hydroxy substituents and/or (aminoalkyl)amino side chains at various positions on the anthracene-9,10-dione nucleus have been studied (Cheng et al., Drugs of the Future, (1983), 8, 229) without notable improvement.

Aza- and diaza anthracene-9,10-diones such as 6,9-bis(ethoxycarbonylamino)benzo[g]quinoline-5,10-dione (1), 6,9-bis(ethoxycarbonylamino)benzo[g]isoquinoline-5,10-dione (2), 6,9-bis(ethoxycarbonylamino)benzo[g]quinazoline-5,10-dione (3), were disclosed by Potts et al. (Synthesis, 1983, 31). These compounds were reported to be related to the antitumor 1,4-bis[(aminoalkyl)amino]anthracene-9,10-diones; however, no antitumor activity data was reported for any of the above compounds. 1-[(aminoalkyl)amino] derivatives (4) of 6,9-dihydroxybenzo[g]isoquinoline-5,10-dione related to mitoxantrone have been disclosed as DNA intercalators but they were completely devoid of any "in vitro" or "in vivo" antitumor activity (Croisy-Delcey et al., Eur. J. Med. Chem., (1988), 23, 101-106).

The 6,9-bis[(aminoalkyl)amino]benzo[g]quinoline-5,10-diones of formulas 5a-d (see Table I) were disclosed in J. Med. Chem. (1985), 28, 1124-26, where they were referred to as 5,8-bis[(aminoalkyl)amino]-1-aza-anthracenediones.

As reported in table II herein below reported the prior art compounds 5 are clearly less active than the corresponding carbocyclic analogues (6).

In fact, as reported in table II, the compounds 5a and 5b are less cytotoxic than the analogues 6a and 6b. Moreover the compound 5a, which is poorly cytotoxic in vitro, is inactive in vivo, and it is both less active and less potent than the carbocyclic analogue 6a.

Even though the introduction of an heteroatom is a common process in the medicinal chemistry, its effect must be evaluated case by case.

In this particular case of aza-analogues of anthracenediones the prior art teachings clearly show that the introduction of a nitrogen atom into the anthracenedione skeleton is detrimental for antitumor activity. In fact the aza-substituted anthracenediones are less cytotoxic than the corresponding anthracenediones and inactive "in vivo".

Thus a person skilled in the art, would have not considered the introduction of heteroatoms into the anthracenedione skeleton as a possible way to obtain more effective antitumor agents.

It is well known that the screening for the discovery of the antitumor agent mitoxantrone (J. Med. Chem., (1978), 21, 291-4) among a number of analogues has been carried out by using the experimental model of murine leukemia P388: this model is then predictive of antitumor activity in humans at least for this class of antitumor antibiotics. Many other clinically active antitumor antibiotics are active on murine leukemias P388 and L1210 such as m-amsacrine and doxorubicin.

Moreover mitoxantrone has shown good activity in other significative experimental tumors such as murine Lewis lung carcinoma and MX1 human mammary carcinoma.

We have now discovered that the compounds of the invention 6,9-bis[(aminoalkyl)amino]benzo[g]isoquinoline-5,10-diones are active as antitumor agents: they are highly effective against murine leukemias L1210 and P388 and against Lewis lung carcinoma and MX1 human mammary carcinoma.

Table I

Prior art compounds

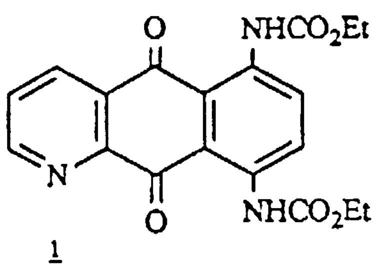

1

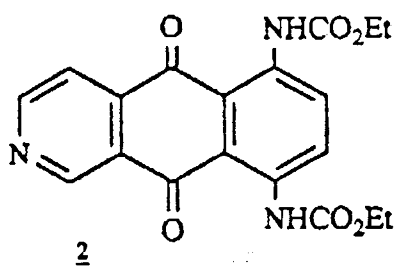

2

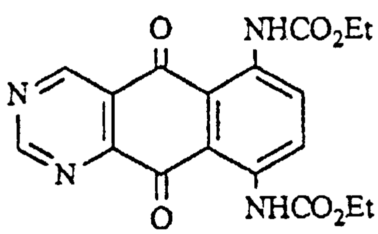

3

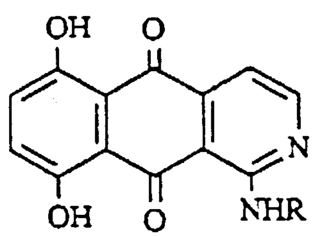

4a R= $CH_2CH_2N(CH_3)_2$

4b R= $CH_2CH_2CH_2N(Et)_2$

4c R= $CH_2CH_2NHCH_2CH_2OH$

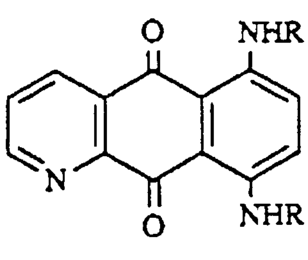

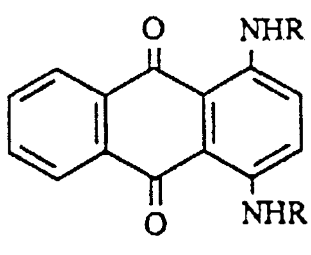

5a R= $CH_2CH_2N(CH_3)_2$

5b R= $CH_2CH_2N(Et)_2$

5c R= $CH_2CH_2CH_2N(CH_3)_2$

5d R= $CH_2CH_2NHCOCH_3$

6a R= $CH_2CH_2N(CH_3)_2$

6b R= $CH_2CH_2N(Et)_2$

Table II

| Antitumor activity of prior art compounds 5 and 6 against murine L 1210 leukemia (from J. Med. Chem. (1985), 28, 1124-1126) | | | |
|---|---|---|---|
| | in vitro | in vivo | |
| | $ID_{50}$ (μg/ml) | Dose (mg/kg) | T/C |
| 5a | 0.16 | 50 | 130 |
| 5b | 2.4 | 100 | toxic |
| 5c | 1.7 | | |
| 6a | 0.08 | 30 | 150 |
| 6b | 0.30 | | |

## BRIEF SUMMARY OF THE INVENTION

The compounds of the invention have the formula (I)

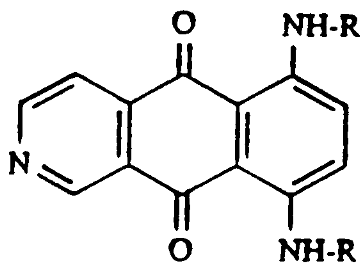

(I)

wherein R is

(a) $-(CH_2)_p-NH_2$ and p is 2, 3 or 4, or
(b) $-(CH_2)_p-NR_2R_3$ and p is 2, 3 or 4 and wherein $R_2$ and $R_3$ are a $C_1$-$C_6$ alkyl or taken together with the nitrogen atom form a heterocyclic ring selected from the group consisting of 1-ethyleneimine, 1-pyrrolidine, 4-morpholine, 1-piperazine, 4-methyl-1-piperazine, 4-benzyl-1-piperazine and 1-piperidine, or
(c) $-(CH_2)_p-NR_2R_3$ and p is 2, 3 or 4 and wherein $R_2$ is hydrogen and $R_3$ is $C_1$-$C_6$ alkyl, or
(d)

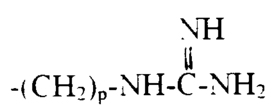

and p is 2, 3 or 4, or
(e) $-(CH_2)_p-NH-(CH_2)q-OH$ and p and q are independently 2, 3 or 4 or
(f) $-(CH_2)_p-OH$ and p is 2, 3 or 4 or
(g) $-(CH_2)_p-O-(CH_2)q-OH$ and p and q are independently 2, 3 or 4 as free bases and their salts with pharmaceutically acceptable acids.

The present invention also concerns the tautomeric forms, the single enantiomers and diastereoisomers of the compounds of formula (I), as well as mixtures thereof.

The present invention also concerns the non-toxic salts of the compounds of formula (I) with acids acceptable for pharmaceutical and veterinary use such as those obtained by addition of inorganic acids like hydrochloric, hydrobromic,

sulfuric, phosphoric, pyrophosphoric acid and/or of organic acids such as acetic, propionic, citric, benzoic, lactic, maleic, fumaric, succinic, tartaric, glutamic, aspartic, gluconic, ascorbic acids and the like.

Specific examples of the preferred compounds of this invention are as follows:

6,9-bis{[(2-(amino)ethyl]amino}benzo[g]isoquinoline-5,10-dione;
6,9-bis{[2-(4'-morpholino)ethyl]amino}benzo[g]isoquinoline-5,10-dione;
6,9-bis{[2-(dimethylamino)ethyl]amino}benzo[g]isoquinoline-5,10-dione;
6,9-bis{[2-(diethylamino)ethyl)amino}benzo[g]isoquinoline-5,10-dione;
6,9-bis{[2-[di(sec-propyl)amino]ethyl]amino}benzo[g]isoquinoline-5,10-dione;
6,9-bis{[2-(1'-pyrrolidino)ethyl]amino}benzo[g]isoquinoline-5,10-dione;
6,9-bis{[2-(1'-aziridino)ethyl]amino}benzo[g]isoquinoline-5,10-dione;
6,9-bis{[2-(methanesulfonyloxy)ethyl]amino}benzo[g]isoquinoline-5,10-dione;
6,9-bis{[4-(amino)butyl]amino}benzo[g]isoquinoline-5,10-dione;
6,9-bis{[3-(amino)propyl)]amino}benzo[g]isoquinoline-5,10-dione;
6,9-bis{[2-[(2-hydroxyethyl)amino]ethyl]amino}benzo[g]isoquinoline-5,10-dione;
6,9-bis{[3-(dimethylamino)propyl]amino}benzo[g]isoquinoline-5,10-dione;
6,9-bis{[(2-hydroxy)ethyl]amino}benzo[g]isoquinoline-5,10-dione;
6,9-bis{[(2-(2-hydroxyethoxy)ethyl]amino}benzo[g]isoquinoline-5,10-dione;
6,9-bis{[2-(methylamino)ethyl]amino}benzo[g]isoquinoline-5,10-dione;
6,9-bis{[2-(ethylamino)ethyl]amino}benzo[g]isoquinoline-5,10-dione;
6,9-bis{[2-(n-propylamino)ethyl]amino}benzo[g]isoquinoline-5,10-dione;
6,9-bis{[2-(sec-propylamino)ethyl]amino}benzo[g]isoquinoline-5,10-dione;
6,9-bis{[2-(guanidino)ethyl]amino}benzo[g]isoquinoline-5,10-dione;
6,9-bis{[(2-amino-2,2-dimethyl)ethyl]amino}benzo[g]isoquinoline-5,10-dione.

The compounds of this invention can be prepared by reaction of 6,9-difluorobenzo[g]isoquinoline-5,10-dione of formula (II)

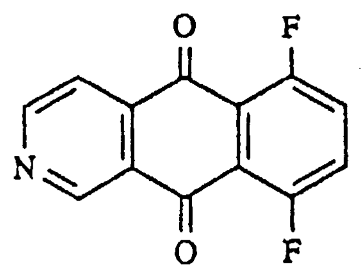

(II)

with a compound of formula (III)

$$R'\text{-}NH_2 \qquad (III)$$

wherein R' has the same meanings as defined in formula (I) for R or it is a group which may be converted to R, to give a compound of formula (Ia):

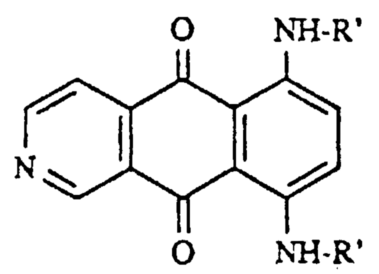

(Ia)

and then optionally performing one or more of the following steps:

a) when R' is other than R, converting R' into R to obtain a compound of formula (I);
b) when R' is one of the groups defined above for R in compounds of formula (I), and in which case the compounds

of formula (Ia) are the same as the compounds of formula (I), R' can be optionally converted into another R group to give another compound of formula (I);

c) optional salification and/or solvation of the obtained compounds of formula (I) or separation of the isomers thereof.

The reaction of the compound of formula (II) with a compound of formula (III) is generally carried out in the presence of a stoichiometric amount or a slight molar excess of a compound of formula (III) in a solvent such as methylene chloride, chloroform, 1,1,1-trichloroethane, dimethoxyethane, tetrahydrofuran, dimethylsulfoxide, dimethylformamide, pyridine, picoline, and mixtures thereof, or, if it is desired, using compound (III) itself as the solvent, optionally in the presence of an inorganic base such as an alkaline or alkaline-earth carbonate or hydrogen carbonate or an organic base such as a trialkylamine, at a temperature from 0°C to the reflux temperature of the solvent.

Preferably the reaction is carried out in a solvent such as pyridine, chloroform or dimethylsulfoxide, using from 2 to 10 equivalents of compound (III) for 1 equivalent of compound (II) and working at a temperature ranging from room temperature to 50°C.

If it is desired the compounds of formula (I) in which R is an hydroxyalkylamino alkyl group of formula $-(CH_2)_p-NH-(CH_2)_q-OH$, wherein p and q are as defined above, can be obtained by reaction of a compound of formula (I) in which R is a group of formula $-(CH_2)_p-OS(O_2)R_5$ with a compound of formula (IV)

$$H_2N-(CH_2)_q-O-E \qquad (IV)$$

wherein E is a hydroxy protective group such as a trialkylsilane, (dialkyl)arylsilane, formyl, acetyl, which reaction may be optionally followed by removal of the protective group E.

If it is desired the compounds of formula (I) in which R is a group of formula $-(CH_2)_p-NR_2R_3$ wherein one of $R_2$ or $R_3$ is hydrogen and the other is hydrogen or a $C_1$-$C_6$ alkyl may be obtained by reaction of compound (II) with a monoprotected diamine of formula (V):

$$H_2N-(CH_2)_p-N(COOR_5)R_3 \qquad (V)$$

wherein $R_3$ is hydrogen or a $C_1$-$C_6$ alkyl, $R_5$ is selected from the group consisting of $C_1$-$C_{10}$ alkyl, $C_7$-$C_{10}$ aralkyl, $\alpha$-, $\beta$- or $\gamma$-naphtyl, phenyl, o-, m- or p-tolyl and p is as defined in formula (I), to give a compound of formula (Ia) in which R' is a group of formula $-(CH_2)_p-N(COOR_5)R_3$, which reaction may be followed by removal of the protective group $COOR_5$.

Useful teachings for the removal of the above mentioned protective groups can be found in Green, T.W., Wuts, P.G.M., "Protective Groups in Organic Syntesis", second Edition, John Wiley and sons, (1991).

The compounds of formula (I) in which R is a group of formula $-(CH_2)_p-NH-C(=NH)NH_2$ can be prepared by reaction of a compound of formula (I) in which R is a group of formula $-(CH_2)_p-NH_2$ with the reagent of formula (VI):

$$H_2N-C(=NH)SO_3H \qquad (VI)$$

for example following the procedure described in Tetra. Lett. (1988), 29, 3183-3186.

The 6,9-difluorobenzo[g]isoquinoline-5,10-dione of formula (II) may be prepared by a multistep procedure involving the Friedel-Crafts acylation of 1,4-difluorobenzene with pyridine-3,4-dicarboxylic acid anhydride, which results in compounds having the structure according to formulas (VIIa) and (VIIb):

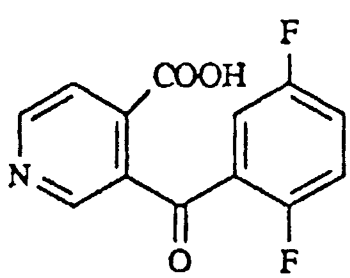

(VIIa)

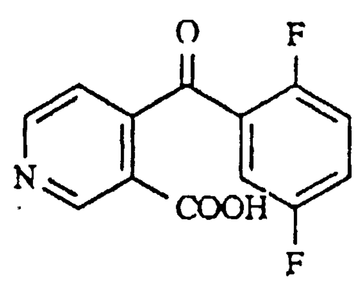

(VIIb)

Compounds according to formulas (VIIa) and (VIIb) may be then subjected to a cyclization reaction in 30% fuming sulfuric acid at approximately 140°C to give the compound of formula (II).

The compounds of formula (III), (IV) and (V) are known, commercially available or they can be prepared accordingly to known procedures.

For example, the compounds of formula (V) may be prepared according to the procedures described in Synth. Comm., (1990), 20, 2559 and in J. Med. Chem., (1990), 33, 97.

The evaluation of the biological activity for the compounds of this invention was performed "in vitro" and "in vivo" following the protocols developed by the U.S. National Cancer Institute.

The evaluation of the "in vitro" cytotoxic activity of the compounds of the invention was performed using a human colon adenocarcinoma cell line (Lovo) isolated from a metastatic nodule and a subline with aquired resistance to a number of antitumor agents, among which doxorubicin, VP-16 and vincristine. This subline (named Lovo/DX) shows reduced accumulation of doxorubicin and overexpression of a protein (Grandi, M., Geroni, C., Giuliani, F.C., British, J. Cancer, (1986), 54, 515). The compounds were tested according to the MTT assay (Mosman, T., "Rapid Colorimetric assay for cellular growth and survival: application to proliferation and cytotoxicity assay", J. Immunol. Methods, (1983), 65, 55-63; Green, L.M., "Rapid colorimetric assay for cell viability; application to the quantitation of cytotoxic and growth inhibitory lymphokines", J. Immunol. Methods, (1984), 70, 257-268) in comparison with mitoxantrone, ametantrone and doxorubicin. The data are reported in table VI.

In general representative compounds of this invention were equally cytotoxic as ametantrone and mitoxantrone in the Lovo cell line, the highest cytotoxicity (superior to that of mitoxantrone) being shown by the compound 6,9-bis{[2-(dimethylamino)ethyl)amino}benzo[g]isoquinoline-5,10-dione (10a) described in example 4 of the experimental section. When ametantrone or mitoxantrone were tested in the Lovo/DX cell line, a resistance index R.I. (defined as the ratio of the $IC_{50}$ for the resistant cell line to the $IC_{50}$ for the sensible cell line) as high as 101.2 and 29.0 respectivelly was found, showing that this subline did have an aquired resistance to mitoxantrone and ametantrone. On the other hand, when representative compounds of this invention were tested in the same resistant subline no cross resistance with mitoxantrone and ametantrone occured, as can be observed for compounds 10a, 12c, 10b, 10e and 10d described in the examples 4, 22, 5, 8 and 7 of the experimental section, respectively. These compounds, in general, showed on this resistant subline the same $IC_{50}$ shown by ametantrone on the sensible Lovo cell line. Compound 10a, in particular, was more active than mitoxantrone in the Lovo/DX cell line.

These "in vitro" data suggest that representative compounds of this invention may be useful in order to overcome the multidrug resistance mediated mechanism of tumor resistance.

The "in vitro" cytotoxic evaluation was also performed on L 1210 murine leukemia cells using the above mentioned cells maintained in suspension cultures (McCoy's 5A medium supplemented with 10% horse serum, glutamine, penicillin and streptomycin) grown in an humidified environment of 10% carbon dioxide and 90% air at 37°C. The compounds were dissolved in dimethylsulfoxide (DMSO) and added to the suspended cells in appropriate concentrations. After 72 hours of continuous exposure, the cell concentration was counted using a Coulter Counter and growth inhibition calculated using the formula:

$$\%\ \text{growth inhibition} = 1 - [\text{cell number treated}/\text{cell number DMSO alone}] \times 100.$$

The $IC_{50}$ was calculated from the growth inhibition data and they are reported in table VII in comparison to the prior art compound 5a (see table I for structure of 5a).

Studies of the biological activity "in vivo" of representative compounds of the invention were performed using the P 388 and L 1210 murine leukemia models.

P 388 murine leukemia cells were intraperitoneally (ip) or intravenously (iv) injected in CP2F1 mice. Treatment was initiated approximately 24 hours after tumor transplantation and dosages of the drug were administered ip (P388 ip/ip) or iv (P388 iv/iv) according to preestablished protocols, usually at 3-day (P388 iv/iv) or 4-day (P388 ip/ip) intervals. The studies were done over a 60-day period and the date of death for each animal was recorded. The % T/C was determined using the mean survival time (MST) for each group according to the formula

$$\%\ T/C = [(\text{MST treated})/(\text{MST control})] \times 100$$

For example two representative compounds of this invention, namely 6,9-bis{[2-(amino)ethyl]amino}benzo[g]isoquinoline-5,10-dione 10i as the dimaleate salt (10i maleate) described in example 12 and 6,9-bis{[(2-dimethylamino)ethyl]amino}benzo[g]isoquinoline-5,10-dione 10a described in example 4 showed an activity superior to that of mitoxantrone in the P 388 iv/iv model. Compound 10i of the present invention, both as the hydrochloride (10i.HCl) and the dimaleate salt (10i.maleate) described in example 12, was superior to mitoxantrone also in the P 388 ip/ip model. Moreover the above representative compounds of the invention showed antileukemic activity over a wide range of well tolerated dosages and in particular they were active at dosages which were lower than the maximum tolerated dose, providing indication for a more favourable therapeutic index in comparison to mitoxantrone. The results are shown in table VIII and table IX.

The antitumor activity of representative compounds of this invention was evaluated also in the L 1210 murine leukemia model.

L 1210 leukemia cells were intraperitoneally (ip) injected in CDFI mice and treatment was initiated approximately 24 hours after tumor transplantation. Dosages of the drugs were administered ip according to preestablished protocols, usually at 4-day intervals. The studies were done over a 60-day period and the date of death for each animal was recorded. The % T/C was determined using the mean survival time (MST) for each group according to the formula

$$\% \, T/C = [(MST \text{ treated})/(MST \text{ control})] \times 100$$

The results are shown in table X and XI

From table X the surprisingly superior antileukemic activity of representative compounds of the invention (namely compound 10a of example 4, compound 10i. HCl of example 12 and 6,9-bis{[2-(2-hydroxyethylamino)ethyl]amino}benzo[g]isoquinoline-5,10-dione 10l prepared in example 17 (dimaleate salt)) in comparison to the prior art compound 5a is evident.

From table XI a clearly favourable comparison again emerges between the excellent antileukemic activity of compound 10i . HCl of the invention and mitoxantrone.

Activity against solid tumors of the compounds of the invention was demonstrated using the murine Lewis Lung carcinoma and the human mammary carcinoma MX-1 models. These tumor models are enclosed in the panel of eight transplanted tumors used by the U.S.-NCI as screen panel for selection of clinically useful antitumor agents (R.K.Y. Zee Cheng and C.C. Cheng, "Screening and evaluation of anticancer agents", Meth. and Find. Expl. Clin. Pharmacol., (1988), 10(2), 67-101). As an example, table XII shows activity data against these two solid tumors of one representative compound of this invention, 6,9-bis{[(2-amino)ethyl]amino}benzo[g]isoquinoline-5,10-dione dimaleate (10i maleate; example 12).

Since representative compounds of this invention show good results against "in vivo" models of murine P 388 and L 1210 leukemias, murine Lewis Lung carcinoma and human mammary carcinoma MX-1, which as noted above are predictive of good results in humans, the compounds disclosed herein are expected to be operative against human leukemias and solid tumors.

The compounds of the present invention may therefore be used as active ingredients of therapeutic compositions to induce regression and/or palliation of cancers in mammals when administered in amounts ranging from about 1 mg to about 0.4 g per kilogram of body weight. A preferred dosage regimen would be from about 1 mg to about 50 mg per kilogram of body weight per day. Unit dosages may be employed so that from about 70 mg to about 3.5 g of the active compound for a subject of about 70 kg of body weight are adminstered in a 24-hour period. The dosage may be adjusted to be compatible to other treatment regimens, such as radiation therapy.

The pharmaceutical composition may be in the form of tablets, capsules, gel capsules, suppositories, lyophilized powders and solutions for intravenous administration.

The invention is illustrated by the following non-limiting examples, and variations which are readily apparent to those skilled in the art. The pertinent formulae are illustrated in the tables I, III, IV and V.

TABLE III

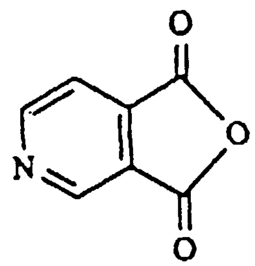

7

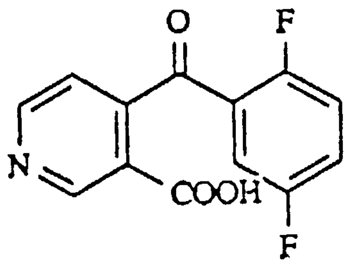

8a

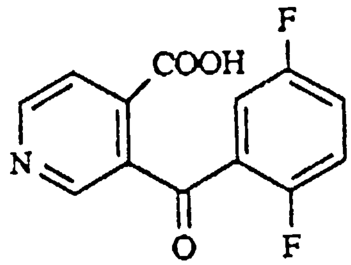

8b

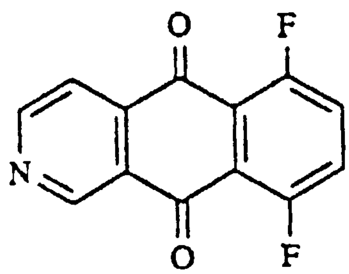

9

TABLE IV

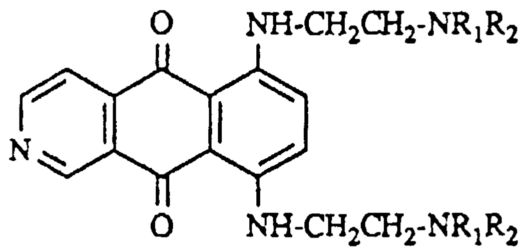

10a, $R = R_1 = CH_3$

b, $R = R_1 = CH_2CH_3$

c, $R = R_1 = CH(CH_3)_2$

d, $R = R_1 = -CH_2CH_2CH_2CH_2-$

e, $R = R_1 = -CH_2CH_2OCH_2CH_2-$

f, $R = R_1 = -CH_2CH_2-$

g, $R = H$, $R_1 = CO_2tBu$

h, $R = CH_3$, $R_1 = CO_2tBu$

i, $R = R_1 = H$

j, $R = H$, $R_1 = COCH_3$

k, $R = H$, $R_1 = CH_3$

l, $R = H$, $R_1 = CH_2CH_2OH$

m, $R = H$, $R_1 = CH_2CH_2OCH_3$

n, $R = H$, $R_1 = CH_2CH_3$

o, $R = H$, $R_1 = CH_2CH_2CH_3$

p, $R = H$; $R_1 = CH(CH_3)_2$

TABLE V

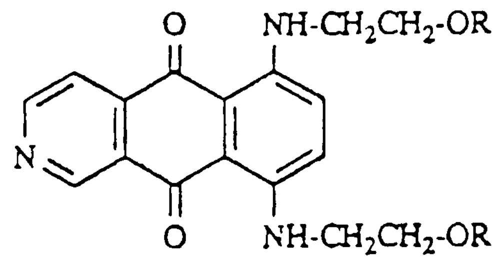

11a, R = H
b, R = $SO_2CH_3$
c, R = $CH_2CH_2OH$

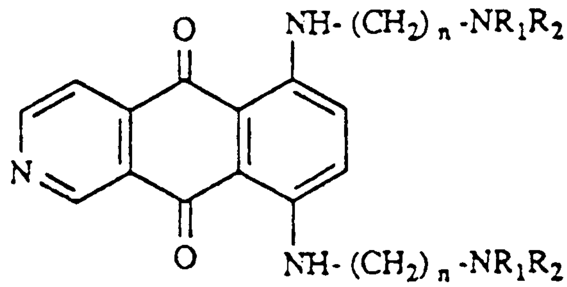

12a, n = 3, $R_1 = R_2$ = H
b, n = 4, $R_1 = R_2$ = H
c, n = 3, $R_1 = R_2 = CH_3$

**Example 1**

Pyridine-3,4-dicarboxylic acid anhydride (7)

A mixture of pyridine-3,4-dicarboxylic acid (15.0 g, 0.09 mol) and acetic anhydride (30 ml) was refluxed for 2 hours. The excess acetic anhydride was removed by distillation and the anhydride was collected and purified by sublimation (123°C at 3 mm Hg) to yield 7 as a white solid (10.1 g, 76%), m.p. 74-76°C. $^1H$ NMR ($CDCl_3$) 9.39 (s, 1H), 9.24 (d, 1H),

7.94 (d, 1H).

**Example 2**

4-(2',5'-Difluorobenzoyl)nicotinic acid (8a) and 3-(2'-5'-difluorobenzoyl)isonicotinic acid (8b)

A mixture of 7 (5.0 g, 0.033 mol) and aluminum chloride (17.5 g, 0.131 mol) in 1,4-difluorobenzene was heated in an oil bath at 110°C for 22 hours. The excess of 1,4-difluorobenzene was recovered by distillation. The residue was cooled in an ice bath and quenched with ice water (75 ml) and conc. hydrochloric acid (6.3 ml). The precipitated solid was filtered and dried to yield a white powder (7.7 g, 87%). This material could be crystallized from acetonitrile and water; m.p. 214-217°C; $^1$H NMR (DMSO-$d_6$), 9.15 (s), 8.90 (d), 8.80 (d), 7.90 (d), 7.5 (m), 7.4 (m). Anal. Calcd for $C_{13}H_7F_2NO_3$: C, 59.32; H, 2.69; N, 5.32. Found: C, 59.03; H, 2.55; N, 5.18.

**Example 3**

6,9-Difluorobenzo[g]isoquinoline-5,10-dione (9)

The keto acid mixture of 8a and 8b (3.0 g, 0.011 mol) in fuming sulfuric acid (7.5 ml, 30% $SO_3$) was heated in an oil bath at 135-140°C for 3 hours. After cooling to room temperature, the mixture was poured into ice (200 ml) and neutralized with sodium bicarbonate. Extraction with methylene chloride gave 9 as a yellow solid (2.0 g, 72%); m.p. 199-200°C; $^1$H NMR ($CDCl_3$) 9.54 (s, 1H), 9.12 (d, 1H), 8.03 (d, 1H), 7.57 (m, 2H). Anal. Calcd. for $C_{13}H_5F_2NO_2$: C, 63.67; H, 2.04; N, 5.71. Found: C, 63.86; H, 1.66; N, 5.39.

**Example 4**

6,9-Bis{[2-(dimethylamino)ethyl]amino}benzo[g]isoquinoline-5,10-dione (10a)

A mixture of 6,9-difluorobenzo[g]isoquinoline-5,10-dione, 9 (20 mg, 0.08 mmol) and N,N-dimethylethylenediamine (0.8 mmol, 0.8 ml of a 1 M solution in pyridine) was stirred at room temperature for 48 hours. Most of the pyridine was allowed to evaporate, ether was added, and the product 10a removed by filtration (24 mg, 79%), m.p. 167-168°C; $^1$H NMR ($CDCl_3$) 11.06 (br t, 1H), 10.97 (br t, 1H), 9.63 (s, 1H), 8.92 (d, 1H), 8.13 (d, 1H), 7.32 (m, 2H), 3.54 (q, 4H), 2.7 (t, 4H), 2.38 (s, 12H); mass spectrum m/z (relative intensity) 381 (2.0, $M^+$), 59 (5.4), 59 (100); U.V. $\lambda$ max (2-methoxyethanol) 580 (sh, 6200), 614 (10,600), 661 (12,700). Anal. Calcd. for $C_{21}H_{27}N_5O_2$: C, 66.12; H, 7.13; N, 18.36. Found: C, 66.22; H, 7.10, N, 18.20.

**Example 5**

6,9-Bis{[2-(diethylamino)ethyl]amino}benzo[g]isoquinoline-5,10-dione (10b)

A solution of N,N-diethylethylenediamine (684 mg, 5.9 mmol) in pyridine (2 ml) was added to 9 (202 mg, 0.82 mmol) in pyridine (2 ml). The purple mixture was stirred at room temperature for 48 hours (TLC analysis at this point showed one blue component: silica gel, 5% MeOH/$CHCl_3$). The excess base and pyridine were removed by evaporation with a slow stream of nitrogen gas. The crude material was then placed under vacuum overnight. Upon addition of ice-water to the residue the solid was collected by filtration and dried (330 mg, 84%); m.p. 142-144°C. $^1$H NMR ($CDCl_3$) 11.10 (t, 1H), 11.00 (t, 1H), 9.60 (s, 1H), 8.92 (d, 1H), 8.10 (d, 1H), 7.25 (m, 3H), 3.50 (m, 1H), 2.82 (m, 1H), 2.35 (m, 3H), 1.10 (m, 12H). Anal. Calcd. for $C_{25}H_{35}N_5O_2$: C, 68.62; H, 16.00; N, 7.31. Found: C, 67.95; H, 16.05; N, 7.28.

**Example 6**

6,9-Bis{[2-[di(sec-propyl)amino]ethyl]amino}benzo[g]isoquinoline-5,10-dione (10c)

N,N-diisopropylethylenediamine (588 mg, 4.1 mmol) was added to compound 9 (100 mg, 0.41 mmol) in methanol (1 ml) and water (1 ml). The mixture was allowed to stir at room temperature for 88 h and then poured into ice water. The blue precipitate was recovered by filtration. The solid was purified by column chromatography over silica gel. The initial eluant was chloroform followed by 2% and 20% methanol in chloroform. Concentration of the latter eluants led to 163 mg (81%) of product 10c. mp 134-136°C; $^1$H NMR ($CDCl_3$): 10.95-11.20 (m, 2H), 9.63 (s, 1H), 8.92 (d, 1H), 8.13 (d, 1H), 7.33 (m, 2H), 3.45 (2q, 4H), 3.10 (h, 4H), 2.82 (t, 4H), 1.08 (d, 24H), Anal. Calcd. for $C_{29}H_{43}N_5O_2$: C, 70.55; H, 8.78; N, 14.19. Found: C, 69.23; H, 8.71; N, 13.43.

**Example 7**

6,9-Bis{[2-(1'-pyrrolidino)ethyl]amino}benzo[g]isoquinoline-5,10-dione (10d)

A solution of 1-(2-aminoethyl)pyrrolidine (690 mg, 6.0 mmol) in pyridine (2 ml) was added to 9 (202 mg, 0.82 mmol) in pyridine (2 ml). The mixture was stirred at room temperature for 49 hours. The excess base and pyridine were removed using a slow stream of nitrogen. The residue was placed under vacuum overnight. Cold water was added to the residue and a tacky solid separated. The product was extracted with chloroform (3 x 35 ml), the extracts dried over sodium sulfate and the chloroform removed by rotary evaporation to yield the title compound (260 mg, 73%); TLC, silica gel, 5% MeOH/$CHCl_3$, one blue spot; m.p. 141-142°C. $^1H$ NMR ($CDCl_3$) 11.15 (t, 1H), 11.00 (t, 1H), 9.65 (s, 1H), 8.90 (d, 1H), 8.05 (d, 1H), 7.35 (m, 2H), 3.65 (m, 4H), 2.90 (m, 4H), 2.70 (m, 8H), 1.85 (m, 8H). Anal. Calcd. for $C_{25}H_{31}N_5O_2$: C, 69.26; H, 7.21; N, 16.15. Found: C, 69.02; H, 7.25; N, 16.00.

Maleate salt. Under a nitrogen atmosphere a solution of maleic acid (271 mg; 2.34 mmol) in ethanol (5 ml) was added to a stirred suspension of compound 10d (450 mg; 1.04 mmol) in ethanol (20 ml). After stirring for two hours diethyl ether (25 ml) was slowly added and the precipitate was filtered and dried under vacuum at 40°C to give the hygroscopic dimaleate salt of 10d (580 mg); m.p. 164-166°C.

Anal. Calcd. for $C_{33}H_{39}N_5O_{10}$ . $H_2O$: C, 57.96; H, 6.04; N, 10.24. Found: C, 57.85, H, 5.99; N 10.14.

**Example 8**

6,9-Bis{[2-(4'-morpholino)ethyl]amino}benzo[g]isoquinoline-5,10-dione (10e)

A solution of 4-(2-aminoethyl)morpholine (220 mg, 1.7 mmol) in pyridine (2 ml) was added to 9 (102 mg, 0.42 mmol) in pyridine (1 ml). The purple mixture was stirred at room temperature for 120 hours. The excess amine and pyridine were evaporated using a slow nitrogen stream and the residue placed under vacuum overnight. The mixture was taken up into chloroform and then filtered. Removal of the chloroform left a blue solid which on TLC (silica gel, 25% MeOH/75% $CHCl_3$/few drops of aq. ammonium hydroxide) showed one blue spot (140 mg, 72%); $^1H$ NMR ($CDCl_3$) 11.015 (m, 1H), 10.90 (m, 1H), 9.62 (d, 1H), 8.90 (d, 1H), 8.10 (d, 1H), 7.25 (m, 2H), 3.80 (m, 8H), 3.55 (m, 4H), 2.75 (t, 4H), 2.50 (m, 8H). Anal. Calcd. for $C_{25}H_{31}N_5O_4$: C, 64.22; H, 6.68; N, 11.98. Found: C, 63.95; H, 6.78; N, 11.89.

**Example 9**

6,9-Bis{[2-(1'-aziridino)ethyl]amino}benzo[g]isoquinoline-5,10-dione (10f)

The procedure of example 4 was repeated reacting compound 9 with N-(2-aminoethyl)aziridine and the residue obtained after evaporation of pyridine was purified by silica gel column chromatography.

A major blue band eluted with 10% $CH_3OH$ in $CHCl_3$ was collected. Crystallization from a mixture of methylene chloride and ligroine yielded a dark blue solid, m.p. 100-103°C; $^1H$ NMR ($CDCl_3$) 11.23 (br, 1H), 11.15 (br, 1H), 9.73 (s, 1H), 8.91 (d, 1H), 8.12 (d, 1H), 7.40 (d, 1H), 7.38 (d, 1H), 3.69 (q, 4H), 2.60 (t, 4H), 1.85 (m, 4H), 1.24 (m, 4H); mass spectrum m/z (relative intensity) 377 (100, $M^+$), 278 (72.7), 56 (12.6). Anal. Calcd. for $C_{21}H_{23}N_5O_2$: C, 66.82; H, 6.14; N, 18.55. Found: C, 66.50; H, 6.00; N, 18.20.

**Example 10**

6,9-Bis{[2-[N-(t-butoxycarbonyl)amino]ethyl]amino}benzo[g]isoquinoline-5,10-dione (10g)

A solution of 9 (0.10 g, 0.41 mmol) and N-(t-butoxycarbonyl)ethylenediamine (Synth. Comm., (1990), 20, 2559; 0.65 g, 4.10 mmol) in pyridine (4.0 ml) was stirred at room temperature for 24 hours. Addition of water (20 ml) led to 10g which was filtered, washed with water and dried (0.17 g, 80%), m.p. 213-216°C. Crystallization from a $CHCl_3$: $CCl_4$ mixture gave a dark blue solid m.p. 215-216°C; $^1H$ NMR ($CDCl_3$) 11.07 (br, 1H), 10.96 (br, 1H), 9.49 (s, 1H), 8.90 (d, 1H), 7.99 (d, 1H), 7.32 (s, 2H), 5.30 (br, 2H), 3.58 (m, 4H), 3.47 (m, 4H), 1.56 (s, 18H); mass spectrum m/z (relative intensity) 525 (100, $M^+$) 339 (51.9), 57 (87.7). Anal. Calcd. for $C_{27}H_{35}N_5O_6$: C, 61.70; H, 6.73; N, 13.32. Found: C, 61.18; H, 6.29; N, 12.88.

**Example 11**

6,9-Bis{[2-(N-t-butoxycarbonyl-N-methylamino)ethyl]amino}benzo[g]isoquinoline-5,10-dione (10h)

6,9-difluorobenzo[g]isoquinoline-5,10-dione (0.29 g, 1.18 mmol) was added to a stirred solution of N-(t-butoxycarbonyl)-N-methylethylenediamine (0.824 g, 4.73 mmol) (J. Med. Chem. 1990, 33, 97) in dry pyridine (5 ml). The reaction mixture was stirred in a nitrogen atmosphere for 24 hours at room temperature and then for further 8 hours at 50°C. The solvent was removed under reduced pressure and the blue residue obtained was taken up with $CH_2Cl_2$ (50 ml), washed with a 5% $NaHCO_3$ solution (2 x 30 ml) and with water (30 ml). The combined organic phases were dried over $Na_2SO_4$ and the solvent evaporated under reduced pressure.

The blue residue was purified by column chromatography (silica gel 230-400 mesh, 50 g, eluent $CH_2Cl_2$: AcOEt: MeOH 93:5:2) yielding 400 mg (61%) of blue crystals, m.p. 161.5 - 162.5°C after recrystallization from $CH_2Cl_2$: hexane. $^1$H NMR ($CDCl_3$) 1.49 (s, 18H); 2.94 (s, 6H); 3.45-3.75 (m, 8H); 7.25-7.55 (m, 2H); 8.12 (d, 1H); 8.95 (d, 1H); 9.62 (s, 1H); 10.95-11.23 (m, 2H, exchangeable with $D_2O$).

## Example 12

6,9-Bis{[2-(amino)ethyl]amino}benzo[g]isoquinoline-5,10-dione (10i)

From difluoride 9: Under a nitrogen atmosphere 1,2-diaminoethane (8.72 ml; 130.5 mmol) was added to a stirred solution of compound 9 (4.00 g, 16.3 mmol) in pyridine (80 ml). A slightly exotermic reaction ensued that was moderated by cooling in a water bath at 20°C. The reaction mixture was stirred at room temperature for 34 hours, then it was cooled to -5°C for 30 minutes, the solid was separated by suction under a nitrogen flow and dried overnight under vacuum at 30°C. The crude material obtained (6.07 g) was recrystallized by dissolution in methanol (20 ml) at 40°C and reprecipitation with methylene chloride (100 ml) and n-hexane (300 ml) to give 5.12 g of 10i as a blue solid.
$^1$H NMR ($CDCl_3$): 11.10-11.30 (2m, 2H), 9.53 (s, 1H), 8.93 (d, 1H)), 8.13 (d, 1H), 7.35 (m, 2H), 3.55 (q, 4H), 3.10 (t, 4H). HPLC Lichrospher 100 RP18, 5 μm; eluent: sodium heptanesulfonate 2 g/l in $H_2O$: $CH_3CN$: dioxane 75:20:5, pH 3 with $H_3PO_4$;$\lambda$ = 245 nm; flow 1 ml/min; $R_T$ = 8.80 minutes.

Maleate salt: Under a nitrogen atmosphere the free base 10i (3.50 g; 10.8 mmol) was dissolved in a mixture of ethanol (170 ml) and methanol (30 ml) by heating at 50°C for 10 minutes, then a solution of maleic acid (2.87 g, 24.7 mmol) in ethanol (30 ml) was added. After heating for five more minutes at 50°C the mixture was allowed to cool to room temperature and then left at 4°C for 2 hours. The precipitate was separated by suction under a nitrogen flow and dried at 40°C under vacuum to give 5.75 g of the crude dimaleate.

To a stirred suspension of this crude material (5.71 g) in water (90 ml) at 50°C ethanol was added dropwise until a complete solution was obtained (about 300 ml), then more ethanol was added (400 ml) and the mixture was allowed to cool to room temperature. After 12 hours the precipitate was separated by suction, washed with ethanol (15 ml) and diethyl ether (10 ml) and then dried under vacuum at 30°C for 5 hours to give 4.22 g of the dimaleate salt of 10i; m.p. 176-178°C shrinking, it does not melt up to 245°C; Anal. Calcd. for $C_{17}H_{19}N_5O_2.2C_4H_4O_4.1.5\ H_2O$: C, 51.37; H, 5.17; N, 11.98. Found: C, 51.31; H 4.99; N 11.93.
$^1$H NMR (DMSO-$d_6$) 10.92 (br, 1H), 10.87 (br, 1H), 9.45 (s, 1H), 9.01 (m, 1H), 8.08 (m, 1H), 7.59 (s, 2H), 6.03 (s, 4H), 3.76 (br, 4H), 3.08 (m, 4H). U.V., 1.528 mg in water (50 ml), nm (E 1%): 641 (271), 597 (245), 313 (98), 273 (257), 246 (479).

Hydrochloride salt:

a) From amine 10i: Hydrogen chloride gas was bubbled through a solution of the crude amine in chloroform until the solution was no longer blue. The solid was filtered and dried to yield a dark blue hygroscopic solid (0.174 g, 26%), m.p. 209-212°C; $^1$H NMR (DMSO-$d_6$) 10.91 (br, 2H), 9,44 (s, 1H), 9.01 (d, 1H), 8.23 (br, 4H), 8.09 (d, 1H), 7.74 (s, 2H), 3.84 (m, 4H), 3.02 (m, 4H). Anal. Calcd. for $C_{17}H_{19}N_5O_2.2HCl.4H_2O$; C, 44.94; H, 6.43; N, 15.41. Found: C, 44.52; H, 5.29; N 14.53. The free amine could be regenerated by adding solid potassium carbonate to the NMR sample. A $^1$H NMR analysis indicated the presence of the free amine.

b) From deprotection of t-Boc Analogue 10g: Hydrogen chloride gas was bubbled through a solution of 10g (0.160 g, 0.30 mmol) in dry chloroform (10 ml) for 30 minutes. A dark blue solid was filtered and dried (0.115 g, 95%); m.p. 213-215°C whose structure was confirmed as the hydrochloride salt of 10i by $^1$H NMR analysis.

## Example 13

6,9-Bis{[2-(acetylamino)ethyl]amino}benzo[g]isoquinoline-5,10-dione (10j)

The procedure of Example 12 using compound 9 and 1,2-diaminoethane, was repeated and the crude reaction mixture was applied to a silica gel column. Acetic anhydride (30 ml) was added to the column and it was allowed to stand for 15 minutes. A major blue fraction 10j eluted with 1:4 $CH_3OH$:$CHCl_3$ which was crystallized from a $CHCl_3$:$CH_3OH$ mixture to yield a blue solid (0.240 g, 35%) m.p. 155-156°C; $^1$H NMR (DMSO-$d_6$) 11.12 (t, 1H), 11.02 (t, 1H), 9.42 (s, 1H), 8.95 (d, 1H), 8.15 (br, 2H), 8.03 (d, 1H), 7.62 (s, 2H), 3.57 (m, 8H), 1.83 (s, 6H); mass spectrum m/z

(relative intensity) 409 (2.5, $M^+$), 337 (6.4), 86 (100). Anal. Calcd. for $C_{12}H_{23}N_5O_4$: C, 61.60; H, 5.67; N, 17.10. Found: C, 61.37; H, 5.42; N, 16.89.

**Example 14**

6,9-Bis{[2-(methylamino)ethyl]amino}benzo[g]isoquinoline-5,10-dione trihydrochloride (10k)

Ethanolic HCl (6.7 N, 2.0 ml) was added to a solution of 6,9-bis{[2-(N-t-butoxycarbonyl-N-methylamino)ethyl]amino}benzo[g]isoquinoline-5,10-dione (10h) (440 mg, 0.795 mmol) in $CHCl_3$ (40 ml). The reaction mixture was stirred at room temperature in a nitrogen atmosphere for 24 hours, then the dark green crystals formed were collected by filtration, washed with absolute ethanol and dried at 40°C in vacuo to give 945 mg (94% yield) of pure product, m.p. 188°C dec. (DSC evaluation).

Elemental Analysis Calcd. for $C_{19}H_{26}Cl_3N_5O_2.2H_2O$: C, 46.51; H, 6.03; N, 14.14; Cl 23.60. Found: C, 45.75; H 6.06; N, 14.04, Cl 21.32. HPLC (Lichrosper 100 RP18, 5 μm; Eluent $K_2HPO_4$ 25 mM, n-$C_7H_{15}SO_3Na$ 2 mg/ml in $H_2O$: $CH_3CN$: dioxane 80:15:5, pH 2.7 with $HClO_4$; λ 275 nm, flow 1 ml/min., $R_T$ 20.11 minutes.
U.V., 1.07 mg in 20.0 ml of water: nm (E 1%): 641 (290); 597 (267); 313 (106); 273 (278); 247 (486).
$^1$H NMR ($D_2O$) 2.75 (s, 6H), 3.30-3.45 (m, 4H) 3.80-3.95 (m, 4H), 7.38 (a, 2H); 8.17 (d, 1H), 8.85 (d, 1H), 9.27 (s, 1H).

**Example 15**

6,9-Bis[(2-hydroxyethyl)amino]benzo[g]isoquinoline-5,10-dione (11a)

Difluoride 9 (1.0 g, 4.1 mmol) and 2-aminoethanol (2.5 g, 40.8 mmol) in pyridine (7 ml) were stirred at room temperature for 18 hours. The mixture was poured into water (50 ml) and the product 11a was filtered and dried (1.26 g, 94%), m.p. 236-239°C. Recrystallization from methanol yielded dark blue needles m.p. 237-239°C; $^1$H NMR (DMSO-$d_6$) 11.28 (t, 1H), 11.19 (t, 1H), 9.43 (s, 1H), 8.94 (d, 1H), 8.03 (d, 1H), 7.56 (s, 2H), 5.01 (t, 2H), 3.69 (m, 4H), 3.54 (m, 4H). Anal. Calcd. for $C_{17}H_{17}N_3O_4$: C, 62.38; H, 5.25; N, 12.83. Found: C, 62.21; H, 5.12; N, 12.50.

**Example 16**

6,9-Bis[(2-methanesulfonyloxyethyl)amino]benzo[g]isoquinoline-5,10-dione (11b)

A solution of 11a (0.30 g, 0.92 mmol) in dry pyridine (4 ml) was stirred at room temperature under nitrogen for 10 minutes. Methanesulfonyl chloride (0.24 g, 2.07 mmol) was added and the mixture stirred for 20 minutes. The mixture was quenched into ice-water (20 ml) and the solid was filtered. Crystallization from a chloroform-ligroine mixture gave a blue solid (0.294 g, 66%); m.p. 116-118°C; $^1$H NMR ($CDCl_3$) 10.98 (br, 2H), 9.59 (s, 1H), 8.97 (d, 1H), 8.09 (d, 1H), 7.38 (d, 1H), 7.34 (d, 1H), 4.50 (t, 4H), 3.83 (q, 4H), 3.09 (s, 6H). Anal. Calcd. for $C_{19}H_{21}N_3O_8S_2$: C, 47.20; H, 4.39; N, 8.69. Found: C, 46.80; H, 4.31; N, 8.48.

**Example 17**

6,9-Bis{[2-[(2-hydroxyethyl)amino]ethyl]amino}benzo[g]isoquinoline-5,10-dione (10l)

a) From mesylate (11b)

A solution of (11b) (0.40 g, 0.83 mmol) and 2-(trimethylsilyloxy)ethylamine (2.21 g, 16.5 mmol) in pyridine (5.0 ml) was stirred at room temperature under a nitrogen atmosphere for 48 hours. The pyridine was removed under a nitrogen flow and the residue was taken up in methylene chloride. This solution was washed with saturated sodium bicarbonate and dried over sodium sulfate. Removal of the solvent left a blue oil which was dried under vacuum overnight. Chromatography on a silica gel column resulted in a cleavage of the Si-O bond to yield 10l. This product was eluted with a 1:5:5 $Et_3N$:$CH_3OH$:$CHCl_3$ mixture. Concentration gave 10l as an oil: $^1$H NMR ($CDCl_3$) 11.19 (br, 1H), 11.06 (br, 1H), 9.46 (s, 1H), 8.83 (d, 1H), 7.98 (d, 1H), 6.98 (s, 2H), 3.82 (m, 4H), 3.52 (m, 4H), 3.07 (m, 4H), 2.96 (m, 4H); U.V. (2-methoxyethanol): 570 (sh, ε = 7200), 612 (ε = 13,200), 658 (ε = 14,000).

b) From difluoride (9)

Under a nitrogen atmosphere 2-[(2-hydroxyethyl)amino]ethylamine, (6.19 ml; 61.2 mmol) was added to a stirred suspension of compound 9 (1.00 g; 4.08 mmol) in pyridine (30 ml) at 0°C.

After one hour the reaction mixture was allowed to reach room temperature and left at this temperature for three

hours. The reaction mixture was applied to a silica gel (100 g) column chromatography and eluted first with $CHCl_3$: $CH_3OH$ 90:10, then with $CHCl_3$:$CH_3OH$ 85:15 and finally with $CHCl_3$:$CH_3OH$:$NH_4OH$ 85:15:1. The fractions containing the product were pooled, the solvents were removed and the residue was subjected to a second purification by silica gel (95 g) column chromatography, eluting with $CHCl_3$:$CH_3OH$:$NH_4OH$ conc. from 95:5:0 to 80:20:2. A final purification over grade I basic alumina (25 g) eluting with $CH_2Cl_2$: EtOH 96:4 gave a residue of 500 mg that was crystallized from a mixture of ethanol (0.5 ml) and methylene chloride (10 ml) to give 10l (142 mg).

Maleate salt: The free amine 10l (138 mg; 0.334 mmol) was dissolved in ethanol (6 ml) under a nitrogen atmosphere and a solution of maleic acid (87 mg; 0.75 mmol) in ethanol (3 ml) was added at room temperature. After cooling for 3 hours at 4°C the blue crystals obtained were separated by suction under a nitrogen flow, washed with ethanol (1 ml) and diethyl ether (2 ml) and dried under vacuum at 40°C to give the dimaleate salt of 10l (160 mg), m.p. 132-133°C; $^1$H NMR (DMSO-$d_6$) 10.94 (br, 1H), 10.88 (br, 1H), 9.47 (s, 1H), 9.02 (d, 1H), 8.60 (br, 2H), 8.09 (d, 1H), 7.63 (s, 2H), 6.01 (s, 4H), 5.32 (br, 2H), 3.89 (m, 4H), 3.71 (m, 4H), 3.26 (m, 4H), 3.12 (m, 4H). U.V. 1.355 mg in $H_2O$ (25 ml); nm (E 1%) : 641 (220), 598 (208), 312 (85), 272 (221), 246 (406).

Anal. Calcd. for $C_{21}H_{27}N_5O_4$. $2C_4H_4O_4$. $H_2O$: C, 52.52; H, 5.57; N, 10.64. Found: C, 52.49; H, 5.56; N, 10.61.

## Example 18

6,9-Bis{[2-(2-methoxyethylamino)ethyl]amino}benzo[g]isoquinoline-5,10-dione (10m)

A solution of 11b (0.15 g, 0.21 mmol) and 2-methoxyethylamine (0.47 g, 6.20 mmol) in pyridine (2.0 ml) was stirred at room temperature under a nitrogen blanket for 48 hours. The piridine and excess amine were removed under vacuum. The residue was dissolved in methylene chloride, washed with a saturated sodium bicarbonate solution and dried over sodium sulfate. Upon removal of the solvent the residue was purified by column chromatography on silica gel by eluting with 1:1 $CH_3OH$:$CHCl_3$. Crystallization from a ethanol and pentane mixture led to 10m (0.091 g, 66%); m.p. 174-175°C; $^1$H NMR ($CDCl_3$) 11.08 (br, 1H), 10.97 (br, 1H), 9.56 (s, 1H), 8.90 (d, 1H), 8.05 (d, 1H), 7.31 (s, 2H), 3.62 (m, 8H), 3.43 (s, 6H), 3.08 (t, 4H), 2.94 (t, 4H), 2.66 (br s, 2H); mass spectrum m/z (relative intensity) 441 (100, $M^+$), 354 (57.4), 266 (50.5). Anal. Calcd. for $C_{23}H_{31}N_5O_4$: C, 62.57; H, 7.09; N, 15.86. Found: C, 62.51; H, 6.92; N, 15.47.

## Example 19

6,9-Bis{[2-(2-hydroxyethoxy)ethyl]amino}benzo[g]isoquinoline-5,10-dione (11c)

A solution of 2-(2-aminoethoxy)ethanol (446 mg, 4.25 mmol) in pyridine (1 ml) was added to compound 9 (100 mg, 0.41 mmol) in pyridine (1 ml). The mixture was stirred at room temperature for 45 h. The excess amine and pyridine were removed via a slow stream of nitrogen gas and the residual solid was placed under vacuum overnight. A cold saturated NaCl solution was added to the solid and the product was extracted with chloroform (6 x 25 ml). The extracts were dried over magnesium sulfate and concentrated on the rotary evaporator to yield 140 mg (82%) of product 11c; mp 97-99°C. $^1$H NMR ($CDCl_3$): 11.15-11.35 (2m, 2H), 9.60 (s, 1H), 8.85 (d, 1H), 8.08 (d, 1H), 7.30 (m, 2H), 3.85 (m, 8H), 3.57-3.75 (2m, 9H), 2.75-3.25 (br s, 1H).

## Example 20

6,9-Bis{[3-(amino)propyl]amino}benzo[g]isoquinoline-5,10-dione (12a)

1) Free Amine: A solution of 1,3-diaminopropane (870 mg, 11.76 mmol) in chloroform (3 ml) was added to 9 (300 mg, 1.22 mmol) in chloroform (6 ml). The purple mixture was allowed to stir for 166 hours at room temperature. The mixture was filtered and the salts washed with chloroform. The solvent was removed by rotary evaporation and the residue was placed under vacuum overnight to yield 290 mg (93%) of 12a; TLC (silica gel, 25% MeOH/75% chloroform/few drops of aq. ammonium hydroxide) indicated one blue spot; m.p. 105°C (softens) 112-115°C. $^1$H NMR (DMSO-$d_6$) 11.15 (m, 1H), 11.05 (m, 1H), 9.45 (s, 1H), 8.90 (d, 1H), 8.0 (d, 1H), 7.55 (br s, 2H), 3.55 (br m, 4H), 4.65 (t, 4H), 3.75 (t, 4H), 1.75 (t, 4H). Anal. Calcd. for $C_{19}H_{23}N_5O_2$: C, 64.57; H, 6.56; N, 19.81. Found: C, 65.00; H, 6.50; N, 19.78.

2) Maleate Salt: A solution of maleic acid (11 mg, 0.86 mmol) in methanol was added to 12a (100 mg, 0.28 mmol) dissolved in methanol (2 ml) and ethyl acetate (2 ml). The addition of more ethyl acetate gave a blue precipitate which was filtered and dried under vacuum (120 mg, 79%), $^1$H NMR (DMSO-$d_6$) 11.10 (t, 1H), 11.00 (t, 1H), 9.45 (s, 1H), 8.95 (d, 1H), 8.05 (d, 1H), 7.75 (br, 4H), 7.60 (s, 2H), 6.0 (s, 4H), 3.60 (m, 4H), 2.95 (t, 4H), 1.95 (m, 4H). Anal. Calcd. for $C_{23}H_{27}N_5O_6$: C, 58.84; H, 5.80; N, 14.92. Found: C, 58.75; H, 5.70; N, 14.85.

**Example 21**

6,9-Bis{[4-(amino)butyl]amino}benzo[g]isoquinoline-5,10-dione (12b)

1) Free Amine: A solution of 1,4-diaminobutane (960 mg, 10.9 mmol) in chloroform (3 ml) was added to 9 (319 mg, 1.3 mmol) in chloroform (6 ml). The purple reaction mixture was stirred at room temperature for 217 hours (9 days). The precipitate salts were removed by filtration amd the filtrate concentrated under a slow nitrogen stream to yield the free amine 12b (400 mg, 80%); TLC (Silica gel, 25% MeOH/75% $CHCl_3$/few drops of aq. ammonium hydroxide); m.p. 80°C, (softens) 90-92°C. $^1H$ NMR (DMSO-$d_6$) 11.15 (m, 1H), 11.05 (m, 1H), 9.42 (s, 1H), 8.90 (d, 1H), 8.0 (d, 1H), 7.5 (br s, 2H), 3.45 (m, 4H), 2.60 (m, 4H), 1.70 (m, 4H), 1.45 (m, 4H). Anal. Calcd. for $C_{21}H_{27}N_5O_2$: C, 66.12; H, 7.13; N, 18.36. Found: C, 64.26; H, 6.95; N, 17.42.

2) Maleate Salt: A solution of maleic acid (116 mg, 1 mmol) in ethyl acetate (4 ml) was added to 12b (124 mg, 0.40 mmol) in a methanol (6 ml) and ethyl acetate (8 ml) solution. A blue oil separated and the mixture was placed in the refrigerator overnight. The solvents were removed by decantation and the remaining solid washed thoroughly with ethyl acetate to yield a blue, very hygroscopic solid, 125 mg (63%). $^1H$ NMR (DMSO-$d_6$) 11.20 (m, 1H), 11.10 (m, 1H), 9.45 (s, 1H), 8.95 (d, 1H), 8.05 (d, 1H), 7.70 (br s, 4H), 6.00 (s, 4H), 3.55 (m, 4H), 2.85 (m, 4H), 1.70 (m, 8H). Anal. Calcd. for $C_{29}H_{35}N_5O_{10}$: C, 56.76; H, 5.75; N, 11.41. Found: C, 53.65; H, 6.10; N, 10.05.

**Example 22**

6,9-Bis{[3-(dimethylamino)propyl]amino}benzo[g]isoquinoline-5,10-dione (12c)

A solution of 3-dimethylaminopropylamine (700 mg, 6.9 mmol) in pyridine (2 ml) was added to compound 9 (100 mg, 0.41 mmol) in pyridine (2 ml). The mixture was stirred at room temperature for 120 h. The excess diamine and pyridine were removed under a slow stream of nitrogen gas and the residue placed under vacuum overnight. The residue was added to cold water and extracted with chloroform (4 x 20 ml). The extract was dried over $Na_2SO_4$ and concentrated by rotary evaporation. The blue solid was purified by column chromatography over silica gel. The initial eluant was 10% methanol/90% chloroform followed by 25% methanol/75% chloroform. The product was eluted by addition of small amounts of ammonium hydroxide to 25% methanol/75% chloroform. Concentration of these eluants led to 92 mg (55%) of the desired product 12c mp 107-109°C. $^1H$ NMR ($CDCl_3$): 11.00-11.20 (2m, 2H), 9.63 (s, 1H), 8.93 (d, 1H), 8.12 (d, 1H), 7.37 (s, 2H), 3.55 (q, 4H), 2.55 (m, 4H), 2.35 (s, 12H), 2.00 (m, 4H).

**Example 23**

6,9-bis{[2-(ethylamino)ethyl]amino}benzo[g]isoquinoline-5,10-dione (10n)

A solution of N-ethylethylenediamine (400 mg, 4.5 mmol) in pyridine (1 ml) was added to compound 9 (98 mg, 0.40 mmol) in pyridine (1 ml). The mixture was stirred at room temperature for 66 h. The pyridine and excess diamine were removed under a slow nitrogen stream and the residual material was placed under vacuum overnight. Chloroform was then added and the chloroform washed twice with cold water. The chloroform extract was dried over $MgSO_4$ and the solvent removed by rotary evaporation. The resultant blue solid was purified by column chromatography over silica gel using 5% methanol/95% chloroform as the initial eluant. The eluant was gradually changed to 5%, 10% and then 50% methanol in chloroform. The desired product was eluted using 50% methanol/48% chloroform/2% ammonium hydroxide. Removal of the solvent led to 32 mg (21%) of product (10n). mp 101-102°C.

$^1H$ NMR ($CDCl_3$) 11.10 (m, 1H), 11.00 (m, 1H), 9.6 (s, 1H), 8.9 (d, 1H), 8.05 (d, 1H), 7.3 (m, 2H), 3.55 (m, 4H), 3.0 (t, 4H), 2.8 (q, 4H), 1.15 (t, 6H).

**Example 24**

6,9-bis{[2-(propylamino)ethyl]amino}benzo[g]isoquinoline-5,10-dione (10o)

A solution of N-propylethylenediamine (403 mg, 4.0 mmol) in pyridine (1 ml) was added to compound 9 (98 mg, 0.40 mmol) in pyridine (1 ml). The reaction mixture was stirred at room temperature for 24 h and concentrated under a slow stream of nitrogen. The residual materaial was placed under vacuum overnight. Ice water was added to the residual and the aqueous layer was extracted with chloroform (5 x 40 ml). The extracts were dried over magnesium sulfate and the chloroform removed by rotary evaporation. The blue solid was purified by column chromatography over silica gel. The initial eluant was 5% methanol/95% chloroform followed by increasing the methanol amounts gradually to 10%, 20%, 30%, 40% and 50%. The desired compound coluld be eluted using 60% methanol/40% chloroform containing some ammonium hydroxide. Removal of the eluant led to 50 mg (30%) of the product (10o). mp 105-106°C.

$^1H$ NMR ($CDCl_3$) 11.15 (m, 1H), 11.05 (m, 1H), 9.6 (s, 1H), 8.9 (d, 1H), 8.10 (d, 1H), 7.3 (m, 2H), 3.6 (m, m, 4H), 3.0 (t, 4H), 2.75 (t, 4H), 1.6 (m, 4H, $H_2O$ peak superimposed), 0.95 (t, 6H).

## Example 25

6,9-bis{[2-(isopropylamino)ethyl]amino}benzo[g]isoquinoline-5,10-dione (10p)

A solution of N-isopropylethylenediamine (450 mg, 4.5 mmol) in pyridine (2 ml) was added to compound 9 (110 mg, 0.45 mmol) in pyridine (1 ml). An instantaneous permanganate-like coloration resulted. The mixture was stirred at room temperature for 40 h. The pyridine and excess diamine were removed under a slow nitrogen stream and the residue was placed under vacuum overnight. Chloroform (20 ml) was added followed by ice water. Since the blue coloration appeared int he aqueous layer, this was extracted with chloroform (4 x 25 ml). The combined extracts were dried over $MgSO_4$ and the chloroform removed by rotary evaporation. The resultant blue solid was purified by column chromatography over silica gel using chloroform as the initial eluant followed by gradual changes to 2%, 5%, 10%, 20%, 40% and 50% methanol in chloroform. The desired product was eluted from the column with 50% methanol/49% chloroform/1% ammonium hydroxide. Removal of the eluants led to 56 mg (30%) of the desired product (10p). mp 136-137°C.
$^1H$ NMR ($CDCl_3$) 11.1 (t, 1H), 11.0 (t, 1H), 9.6 (s, 1H), 8.9 (d, 1H), 8.1 (d, 1H), 7.30 (m, 2H), 3.6 (m, 4H), 3.0 (t, 4H), 2.9 (m, 2H), 1.05 (d, 6H).

## Example 26

In vitro biological evaluation: MTT assay on human colon adenocarcinoma Lovo cells.

The MTT assay was performed according to Mosmann, T., J. Immunol. Methods, (1983), 65, 55-63 and Green, L. M., J. Immunol. Methods, (1984), 70, 257-268.

Immediately before use, compounds and reference standards are dissolved in proper solvent and subsequently further diluted in complete culture medium. Human colon adenocarcinoma cells Lovo and the subline resistant to doxorubicin (Lovo/DX) (2.5. $10^4$ cells/ml) are plated in 96-well plates and preincubated for 24 hours in culture medium. After this time, the cells are exposed to drugs for 144 hours, then the thiazolyl blue tetrazolium bromide solution (MTT solution) is added and the cells are reincubated for 4 hours. The supernatant is removed and 150 μl of dimethylsulfoxide are added to solubilize formazan crystals. The plate is read at 570 nm with microplate reader and the inhibiting concentration of 50% cellular growth ($IC_{50}$; μg/ml) is calculated.

The results are reported in Table VI

TABLE VI

IN VITRO CYTOTOXIC ACTIVITY OF REPRESENTATIVE COMPOUNDS OF THE INVENTION ON LOVO AND LOVO/DX CELLS BY MTT ASSAY

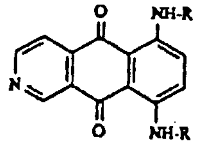

| COMPOUND | EXAMPLE | R = | IC50 µg/ml±S.D. a) | | |
|---|---|---|---|---|---|
| | | | LoVo | LoVo/Dx | R.I.b) |
| 10a | 4 | 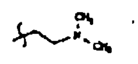 | 0.045 | 0.085 | 1.9 |
| 12c | 22 | 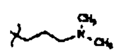 | 0.15±0.07 | 0.36±0.04 | 2.4 |
| 10b | 5 | 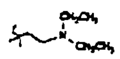 | 0.29±0.05 | 0.69±0.1 | 2.4 |
| 10e | 8 | 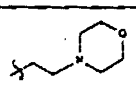 | 5.32±1.55 | 2.29±0.67 | 0.4 |
| 10d | 7 | 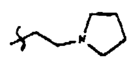 | 0.20±0.03 | 0.52±0.15 | 2.6 |
| 10i (maleate) | 12 | 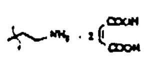 | 0.21±0.08 | 6.70±1.8 | 31.8 |
| 12a | 20 | 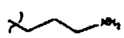 | 1.64±0.02 | 20.9±0.72 | 12.7 |
| Mitoxantrone | | | 0.01±0.009 | 0.29±0.1 | 29.0 |
| Ametantrone | | | 0.33±0.1 | 33.4±13.9 | 101.2 |
| Doxorubicin | | | 0.03±0.009 | 4.28±1.8 | 142.7 |

a) MTT assay. 144 hrs drug exposure.
b) R.I. = (IC50 LoVo/Dx)/(IC50 LoVo).

## Example 27

In vitro biological evaluation: L1210 murine leukemia

L1210 Murine leukemia cells were routinely maintained in suspension cultures in McCoy's 5A medium supplemented with 10% horse serum glutamine, penicillin and streptomycin and grown in a humidified environment of 10% carbon dioxide and 90% air at 37°C.

To assess the in vitro toxicity, each compound was dissolved in dimethylsulfoxide and added to 1 ml of L1210 cells ($10^5$ cells/tube) to attain final concentrations of 0.01, 0.1 and 1 μg of drug/ml of culture. After 72 hours of continues exposure to the drug, the cell concentration was determined with a Coulter Counter. Growth inhibition was calculated for each drug using the following formula:

% growth inhibition = 1-[cell number treated/cell number DMSO alone] x 100.

The growth inhibition data was then used to calculate the $IC_{50}$ value (the calculated drug concentration required to inhibit cell growth by 50% of control.

The results are reported in Table VII

TABLE VII

| IN VITRO CYTOTOXIC ACTIVITIES OF REPRESENTATIVE COMPOUNDS OF THE INVENTION AGAINST L1210 LEUKEMIA IN COMPARISON TO THE PRIOR ART COMPOUND 5a | | |
|---|---|---|
| Compound | Example | $IC_{50}$ (μg/ml) |
| 10a | 4 | 0.006 |
| 10f | 9 | 0.002 |
| 10i[(a)] | 12 | 0.05 |
| 10l[(b)] | 17 | 0.06 |
| 5a[(c)] | / | 0.155 |

(a) Hydrochloride Salt: 10i.HCl
(b) Dimaleate Salt: 10l.maleate
(c) Prior art compound, 6,9-bis{[2-(dimethylamino)ethyl]amino}benzo[g]quinoline-5,10-dione: A.P. Krapcho et al., J. Med. Chem. (1985), 28, 1124-1126.

## Example 28

In vivo biological studies: P388 Murine Leukemia (iv/iv, d 1, 4, 7)

P388 Murine Leukemia cells were maintained in vivo by serial intraperitoneal (i.p.) injections of $10^6$ cells in DBA2 mice. For test purposes, CDF1 mice were inoculated intravenously (i.v.) with $10^6$ P388 cells and treatment was initiated 24 hr later. The i.v. dose of drug was administered on days 1, 4 and 7. Mice were observed daily for signs of toxicity and survival. The date of death was recorded for each animal that died or was sacrificed during the 60 day study. The median survival time (MST) for each treatment group was calculated and the % T/C was determined using the following formula:

% T/C = [(MST treated)/(MST control)] x 100

The results of the antileukemic activity of 6,9-bis{[2-(dimethylamino)ethyl]amino}benzo[g]isoquinoline-5,10-dione (10a) of example 4 and 6,9-bis{[2-(amino)ethyl]amino}benzo[g]isoquinoline-5,10-dione dimaleate (10i maleate) of example 12 in comparison to mitoxantrone are reported in table VIII

Table VIII

| ANTITUMOR ACTIVITY OF COMPOUNDS 10a, 10i.maleate AND MITOXANTRONE (MITOX) AGAINST P388 MURINE LEUKEMIA (iv/iv 1,4,7)[1] | | | | |
|---|---|---|---|---|
| COMPOUND[2] | DOSE (mg/kg/day) | T/C%[3] (range) | TOX[4] | LTS[5] |
| Controls | - | 100 | 0/73 | 0/73 |
| 10i.maleate | 18 | 200,188 | 0/16 | 0/16 |
| | 27 | 203(181-227) | 2/32 | 0/32 |
| 10a | 18 | 172,156 | 0/14 | 0/14 |
| | 27 | 188,167 | 3/17 | 0/17 |
| | 40 | 183,156 | 1/16 | 0/16 |
| MITOX | 2 | 162(154-167) | 0/42 | 0/42 |
| | 3 | 185(167-217) | 4/78 | 0/78 |
| | 4 | 118(89-133) | 20/24 | 0/24 |

1) CDF1 male mice were transplanted iv with $10^6$ cells/mouse. Treatment was given iv on days 1,4,7 after tumor transplantation (day 0).
2) 10i.maleate and MITOX were dissolved in sterile water; 10a in citric acid 1%.
3) Median survival time of treated mice/median survival time of controls x 100:
4) Number of toxic deaths/total number of mice.
5) Long term survivors: animals tumor free at the end of the experiment (60 days).

**Example 29**

In vivo biological studies: P388 Murine Leukemia (ip/ip, d 1, 5, 9)

P388 Murine Leukemia cells were maintained in vivo by serial intraperitoneal (ip) injections of $10^6$ cells in DBA2 mice. For test purposes, CDF1 mice were inoculated i.p. with $10^6$ P388 cells and treatment was initiated 24 hours later. The i.p dose of drug was administered on days 1, 5 and 9. Mice were observed daily for signs of toxicity and survival. The date of death was recorded for each animal that died or was sacrificed during the 60-day study. The median survival time (MST) for each treatment group was calculated and the % T/C was determined using the following formula:

$$\% T/C = [(MST\ treated)/(MST\ control)] \times 100$$

The results of the antileukemic activity of 6,9-bis{[2-(amino)ethyl]amino}benzo[g]isoquinoline-5,10-dione (10i) as the dihydrochloride (10i.HCl) and dimaleate (10i.maleate) salts (example 12) in comparison to mitoxantrone are reported in table IX

Table IX

| ANTITUMOR ACTIVITY OF <u>10i</u>.HCl, <u>10i</u>.maleate AND MITOXANTRONE (MITOX) AGAINST ASCITIC P388 MURINE LEUKEMIA (ip/ip 1,5,9)[1)] | | | | |
|---|---|---|---|---|
| COMPOUNDS[2)] | DOSE (mg/kg/day) | % T/C[3)] (range) | TOX[4)] | LTS[5)] (day 60) |
| 10i.HCl | 3.1 | 144 | 0/8 | 0/8 |
| | 6.25 | 196 | 1/8 | 0/8 |
| | 12.5 | 239 | 0/8 | 0/8 |
| | 25 | 367 | 0/8 | 2/8 |
| 10i.maleate | 25 | 185 | 0/8 | 0/8 |
| | 37.5 | 260 | 1/8 | 1/8 |
| MITOX | 1.5 | 186(160-225) | 6/26 | 1/26 |
| | 3 | 173(142-205) | 21/42 | 1/42 |

1) CDF1 mice were injected ip with $10^6$ cells/mouse; treatment was given ip on days 1,5,9 after tumor transplantation (day 0)
2) 10i.HCl and 10i.maleate were dissolved in sterile water and MITOX in saline.
3) Median survival time of treated mice/median survival time of controls x 100
4) No. of toxic deaths/ total No. of mice
5) Long term survivors at the end of the experiment (60 days).

**Example 30**

In vivo biological studies: L1210 Murine Leukemia (ip/ip, d 1, 5, 9)

a) L1210 murine leukemia cells were maintained in vivo by weekly intraperitoneal (ip) injections of $10^6$ cells in $BDF_1$ mice. For test purposes, mice were inoculated i.p. with $10^6$ L1210 cells and treatment was initiated 24 hours later. The desired dose of drug was administered on days 1, 5 and 9. Mice were observed daily for signs of toxicity and survival. The date of death was recorded for each animal that died or was sacrificed during the 60-day study. The mean survival time (MST) for each treatment group was calculated and the % T/C was determined using the following formula:

$$\% T/C = [(MST\ treated)/(MST\ control)] \times 100$$

The results are reported in table X

b) L1210 murine leukemia cells were maintained in vivo by weekly intraperitoneal (ip) injections of $10^5$ cells in DBA2 mice. For test purposes, CDF1 mice were inoculated i.p. with $10^5$ L1210 cells and treatment was initiated 24 hours later. The desired dose of drug was administered on days 1, 5 and 9. Mice were observed daily for signs of toxicity and survival. The date of death was recorded for each animal that died or was sacrificed during the 60-day study. The median survival time (MST) for each treatment group was calculated and the % T/C was determined using the following formula:

$$\% T/C = [(MST\ treated)/(MST\ control)] \times 100$$

The results are reported in table XI

TABLE X

| IN VIVO ANTITUMOR ACTIVITIES OF COMPOUNDS 10a, 10i . HCl AND 10l AGAINST L1210 LEUKEMIA (ip/ip, d 1, 5, 9) IN COMPARISON TO THE PRIOR ART COMPOUND 5a. | | | | |
|---|---|---|---|---|
| Compound | Example | Dose[a] | %T/C | LTS[f] |
| 10a[(b)] | 4 | 50 | 151 | - |
| 10i.HCl[(c)] | 12 | 25 | 265 | 5/6 |
| | | 12.5 | 265 | 4/6 |
| 10l.maleate[(d)] | 17 | 25 | 415 | 1/6 |
| | | 12.5 | 313 | 2/6 |
| 5a[(e),(b)] | / | 50 | 129 | - |

(a) mg/kg, days 1, 5 and 9
(b) Compound 10a and 5a were dissolved in DMSO (<35%) and water
(c) Hydrochloride salt dissolved in water
(d) Dimaleate salt dissolved in water
(e) Prior art compound, 6,9-bis{[2-(dimethylamino)ethyl]amino}benzo[g]quinoline-5,10-dione: A.P. Krapcho et al., J. Med. Chem., (1985), 28, 1124-1126.
(f) Long Term Survivors: animals tumor-free at the end of the experiment (60 days)

Table XI

| ANTITUMOR ACTIVITY OF COMPOUND 10i.HCl AND MITOXANTRONE (MITOX) AGAINST ASCITIC MURINE LEUKEMIA L1210 (ip/ip d.1,5,9)[1)] | | | | |
|---|---|---|---|---|
| COMPOUNDS[2)] | DOSE (mg/kg/day) | % T/C[3)] | TOX[4)] | LTS[5)] |
| 10i.HCl | 6.25 | 156 | 0/8 | 0/8 |
| | 12.5 | 175 | 0/8 | 1/8 |
| | 25 | 250* | 1/7 | 4/7 |
| MITOX | 1.5 | 188 | 0/8 | 1/8 |
| | 3 | 194 | 1/8 | 1/8 |

1) CDF1 mice were injected ip with $10^5$ cells/mouse; treatment was given ip on days 1,5,9 after tumor transplantation (day 0).
2) 10i.HCl was dissolved in sterile water and MITOX in saline.
3) Median survival time of treated mice/median survival time of controls x 100
4) No. of toxic deaths/ total No. of mice at the end of the experiment (60 days).
5) Long term survivors: animals tumor free at the end of the experiment ( 60 days).
* This value was calculated on dead mice.

**Example 31**

In vivo biological studies: antitumor activity against murine Lewis Lung carcinoma and human MX-1 mammary carcinoma

a) Murine Lewis Lung carcinoma

C57b1/6 female mice were trasplantated im (intralimbs) with $10^5$ cells. Treatment was given iv (intravenously) on days 1, 7, 15, after tumor transplantation (day 0). The mean tumor weight for each treatment group was calculated according to Geran, R.I. et al., Cancer Chemother. Rep., (1972), 3, 51-61 and the TWI% was calculated 7 days after the last drug treatment using the formula:

TWI% = 100-[(Mean tumor weight of treated mice)/(mean tumor weight of controls)] x 100.

The results are reported in table XII for the representative compound of the invention 6,9-bis{[2-(amino)ethyl]amino}benzo[g]isoquinoline-5,10-dione (10i) as the dimaleate salt (10i maleate) of the example 12.

b) Human MX-1 mammary carcinoma

CD1 nu/nu female mice were transplantated sc (subcutaneously) with tumor fragments (about 1 mm x 1 mm x 1 mm). Treatment was given iv once a week for three weeks, when tumor weight reached an average of 150 mg. For all individual tumors the weight change (relative tumor weight) for the start of treatment ($V_o$) was expressed as $V_t/V_o$ at each day of measurement ($V_t$). TWI% was calculated 7 days after the last drug treatment using the formula:

TWI% = 100-[(mean relative tumor weight of treated mice)/(mean relative tumor weight of controls)] x 100.

The results are reported in table XII for the representative compound of the invention 6,9-bis{[2-(amino)ethyl)amino}benzo[g]isoquinoline-5,10-dione (10i) as the dimaleate salt (10i maleate) of the example 12.

TABLE XII

| ACTIVITY ON SOLID TUMORS OF 10i MALEATE | | | | | |
|---|---|---|---|---|---|
| TUMOR MODEL AND TREATMENT SCHEDULE | COMPOUND | OPTIMAL DOSE (mg/Kg/day) | T/C % a) | TWI % b) | TOX c) |
| LEWIS LUNG im/iv 1,8,15 murine lung carcinoma | 10i maleate | 40 | 158 | 80 | 0/10 |
| | | 60 | 182 | 95 | 1/10 |
| MX-1 sc/iv q 7d x 3 human mammary carcinoma | 10i maleate | 17 | - | 65 | 0/7 |
| | | 26.6 | - | 58 | 0/7 |
| | | 40 | - | 64 | 1/5 |

a) Median survival time of treated mice/median survival time of controls x 100.
b) Percent of tumor Weight Inhibition.
c) Number of toxic deaths/total number of mice.

## Claims

1. A compound according to formula I

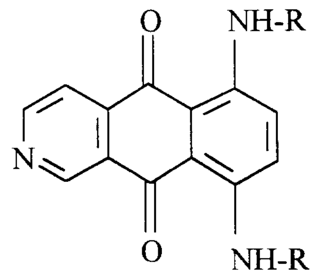

(I),

wherein R is

(a) $-(CH_2)_p-NH_2$ and p is 2, 3 or 4, or

(b) $-(CH_2)_p-NR_2R_3$ and p is 2, 3 or 4 and wherein $R_2$ and $R_3$ are a $C_1$-$C_6$ alkyl or taken together with the nitrogen atom form a heterocyclic ring selected from the group consisting of 1-ethyleneimine, 1-pyrrolidine, 4-morpholine, 1-piperazine, 4-methyl-1-piperazine, 4-benzyl-1-piperazine and 1-piperidine, or

(c) $-(CH_2)_p-NR_2R_3$ and p is 2, 3 or 4 and wherein $R_2$ is hydrogen and $R_3$ is $C_1$-$C_6$ alkyl, or

(d)

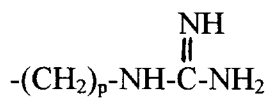

and p is 2, 3 or 4, or

(e) $-(CH_2)_p-NH-(CH_2)_q-OH$ and p and q are independently 2, 3 or 4 or

(f) $-(CH_2)_p-OH$ and p is 2, 3 or 4 or

(g) $-(CH_2)_p-O-(CH_2)q-OH$ and p and q are independently 2, 3 or 4 as free bases and their salts with pharmaceutically acceptable acids.

**2.** A compound according to claim 1 selected from the group consisting of:

6,9-bis([2-(amino)ethyl]amino)benzo[g]isoquinoline-5,10-dione;
6,9-bis([2-(4'-morpholino)ethyl]amino)benzo[g]isoquinoline-5,10-dione;
6,9-bis([2-(dimethylamino)ethyl]amino)benzo[g]isoquinoline-5,10-dione;
6,9-bis([2-(diethylamino)ethyl]amino)benzo[g]isoquinoline-5,10-dione;
6,9-bis([2-[di(sec-propyl)amino]ethyl]amino)benzo[g]isoquinoline-5,10-dione;
6,9-bis([2-(1'-pyrrolidino]ethyl]amino)benzo[g]isoquinoline-5,10-dione;
6,9-bis([2-(1'-aziridino)ethyl]amino)benzo[g]isoquinoline-5,10-dione;
6,9-bis([2-methanesulfonyloxy)ethyl]amino)benzo[g]isoquinoline-5,10-dione;
6,9-bis([4-(amino)butyl]amino)benzo[g]isoquinoline-5,10-dione;
6,9-bis([3-(amino)propyl]amino)benzo[g]isoquinoline-5,10-dione;
6,9-bis([2-[(2-hydroxyethyl)amino]ethyl]amino)benzo[g]-isoquinoline-5,10-dione;
6,9-bis([3-(dimethylamino)propyl]amino)benzo[g]isoquinoline-5,10-dione;
6,9-bis([(2-hydroxy)ethyl]amino)benzo[g]isoquinoline-5,10-dione;
6,9-bis([(2-(2-hydroxyethoxy)ethyl]amino)benzo[g]isoquinoline-5,10-dione;
6,9-bis([2-(methylamino)ethyl]amino)benzo[g]isoquinoline-5,10-dione;
6,9-bis([2-(ethylamino)ethyl]amino)benzo[g]isoquinoline-5,10-dione;
6,9-bis([2-(n-propylamino)ethyl]amino)benzo[g]isoquinoline-5,10-dione;
6,9-bis([2-(sec-propylamino)ethyl]amino)benzo[g]isoquinoline-5,10-dione;
6,9-bis([2-(guanidino)ethyl]amino)benzo[g]isoquinoline-5,10-dione;
6,9-bis([(2-amino-2,2-dimethyl)ethyl]amino)benzo[g]isoquinoline-5,10-dione.

**3.** 6,9-bis([2-(acetylamino)ethyl]amino)benzo[g]isoquinoline-5,10-dione as a free base and its salts with pharmaceutically acceptable acids.

**4.** A compound according to claim 1 which is 6,9-bis([2-(amino)ethyl]amino)benzo[g]isoquinoline-5,10-dione or a salt

thereof.

5. The compound according to claim 1, wherein said salt is a dimaleate salt.

6. A pharmaceutical composition comprising a compound according to claim 1 - 5 and a pharmaceutically acceptable diluent or excipient.

7. A pharmaceutical composition comprising 6,9-bis([2-(amino)ethyl]amino]amino)benzo[g]isoquinoline-5,10-dione or a salt thereof and a pharmaceutically acceptable diluent or excipient.

8. The pharmaceutical composition according to claim 7, wherein said salt is a dimaleate salt.

9. Use of a compound according to claims 1 - 5 for the production of a medicament for the treatment of tumors in a mammal requiring such treatment.

10. A process for the synthesis of a compound according to formula I

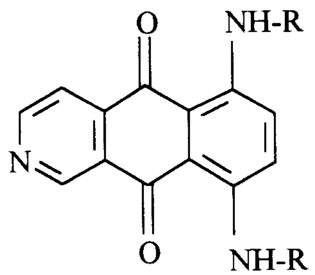

(I),

wherein R is

(a) $-(CH_2)_p-NH_2$ and p is 2, 3 or 4, or
(b) $-(CH_2)_p-NR_2R_3$ and p is 2, 3 or 4 and wherein $R_2$ and $R_3$ are a $C_1$-$C_6$ alkyl or taken together with the nitrogen atom form a heterocyclic ring selected from the group consisting of 1-ethyleneimine, 1-pyrrolidine, 4-morpholine, 1-piperazine, 4-methyl-1-piperazine, 4-benzyl-1-piperazine and 1-piperidine, or
(c) $-(CH_2)_p-NR_2R_3$ and p is 2, 3 or 4 and wherein $R_2$ is hydrogen and $R_3$ is $C_1$-$C_6$ alkyl, or
(d)

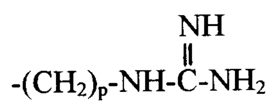

and p is 2, 3 or 4, or
(e) $-(CH_2)_p-NH-(CH_2)q-OH$ and p and q are independently 2, 3 or 4 or
(f) $-(CH_2)_p-OH$ and p is 2, 3 or 4 or
(g) $-(CH_2)_p-O-(CH_2)q-OH$ and p and q are independently 2, 3 or 4 as free bases and their salts with pharmaceutically acceptable acids, comprising the steps of

a) reacting pyridine -3,4-dicarboxylic anhydride with 1,4-difluorobenzene;
b) cyclizing the product of step a) in fuming sulfuric acid at elevated temperature; and
c) displacement of fluoride by addition of amine of formula $R'-NH_2$, wherein R' is the same as that defined by R.

11. The process according to claim 10, wherein a compound obtained in step (c) has the formula (Ia):

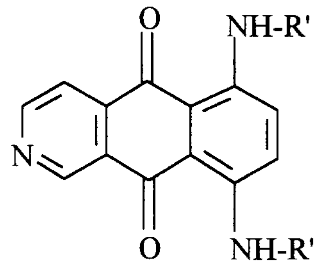

(Ia),

and wherein R' has a meaning other than that defined by R and further comprising the step of converting R' to R.

**12.** The process according to claim 10, further comprising the step of converting R' in the compound obtained in step (c) to another compound defined by R.

**13.** The process according to claim 10, further comprising the step of subjecting the compound obtained in step (c) to salification and/or solvation or separating isomers thereof.

**14.** A process for the synthesis of a compound according to formula I of claim 10, wherein R is

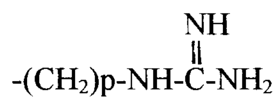

and p is 2, 3 or 4 by reacting a compound of formula I wherein R is $-(CH_2)p-NH_2-$ and p is 2, 3 or 4 with a compound having the formula $H_2N-C(=NH)SO_3H$.

**15.** A process for the synthesis of the compound of claim 3 by reacting 6,9-bis ([2-(amino)ethyl)amino]benzo[g]isoquinoline-5,10-dione obtained according to the process of claim 10 with acetic anhydride.

**Patentansprüche**

**1.** Verbindung gemäß Formel I

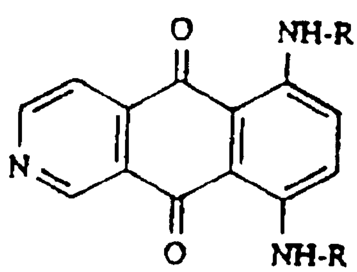

(I),

worin R

(a) $-(CH_2)_p-NH_2$ ist, und p 2, 3 oder 4 ist, oder
(b) $-(CH_2)_p-NR_2R_3$ ist, und p 2, 3 oder 4 ist, und worin $R_2$ und $R_3$ ein $C_1$-$C_6$-Alkyl sind oder zusammen mit dem Stickstoff-Atom einen heterocyclischen Ring bilden, ausgewählt aus der Gruppe bestehend aus 1-Ethylenimin, 1-Pyrrolidin, 4-Morpholin, 1-Piperazin, 4-Methyl-1-piperazin, 4-Benzyl-1-piperazin und 1-Piperidin, oder
(c) $-(CH_2)_p-NR_2R_3$ ist, und p 2, 3 oder 4 ist, und worin $R_2$ Wasserstoff ist und $R_3$ $C_1$-$C_6$-Alkyl ist, oder
(d)

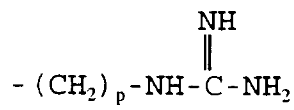

ist, und p 2, 3 oder 4 ist, oder

(e) $-(CH_2)_p-NH-(CH_2)_q-OH$ ist, und p und q unabhängig voneinander 2, 3 oder 4 sind, oder
(f) $-(CH_2)_p-OH$ ist, und p 2, 3 oder 4 ist, oder
(g) $-(CH_2)_p-O-(CH_2)_q-OH$ ist, und p und q unabhängig voneinander 2, 3 oder 4 sind,

als freie Base und deren Salze mit pharmazeutisch annehmbaren Säuren.

**2.** Verbindung nach Anspruch 1, ausgewählt aus der Gruppe bestehend aus

6,9-Bis[(2-aminoethyl)amino]benzo[g]isochinolin-5,10-dion;
6,9-Bis{(2-(4'-morpholino)ethyl]amino}benzo[g]isochinolin-5,10-dion;
6,9-Bis{[2-(dimethylamino)ethyl]amino}benzo[g]isochinolin-5,10-dion;
6,9-Bis{[2-(diethylamino)ethyl]amino}benzo[g]isochinolin-5,10-dion;
6,9-Bis{[2-[di(s-propyl)amino]ethyl]amino}benzo[g]isochinolin-5,10-dion;
6,9-Bis{[2-(1'-pyrrolidino)ethyl]amino}benzo[g]isochinolin-5,10-dion;
6,9-Bis{[2-(1'-aziridino)ethyl]amino}benzo{g]isochinolin-5,10-dion;
6,9-Bis{[2-(methansulfonyloxy)ethyl]amino}benzo[g]isochinolin-5,10-dion;
6,9-Bis[(4-aminobutyl)amino]benzo[g]isochinolin-5,10-dion;
6,9-Bis[(3-aminopropyl)amino]benzo[g]isochinolin-5,10-dion;
6,9-Bis{[2-[(2-hydroxyethyl)amino]ethyl]amino}benzo[g]isochinolin-5,10-dion;
6,9-Bis{[3-(dimethylamino)propyl]amino}benzo[g]isochinolin-5,10-dion;
6,9-Bis{[(2-hydroxy)ethyl]amino}benzo[g]isochinolin-5,10-dion;
6,9-Bis{[2-(2-hydroxyethoxy)ethyl]amino}benzo[g]isochinolin-5,10-dion;
6,9-Bis{[2-(methylamino)ethyl]amino}benzo[g]isochinolin-5,10-dion;
6,9-Bis{[2-(ethylamino)ethyl]amino}benzo[g]isochinolin-5,10-dion;
6,9-Bis{[2-(n-propylamino)ethyl]amino}benzo[g]isochinolin-5,10-dion;
6,9-Bis{[2-(s-propylamino)ethyl]amino}benzo[g]isochinolin-5,10-dion;
6,9-Bis[(2-guanidinoethyl)amino]benzo[g]isochinolin-5,10-dion;
6,9-Bis{[(2-amino-2,2-dimethyl)ethyl]amino}benzo[g]isochinolin-5,10-dion.

**3.** 6,9-Bis{[2-(acetylamino)ethyl]amino}benzo[g]isochinolin-5,10-dion als freie Base und deren Salze mit pharmazeutisch annehmbaren Säuren.

**4.** Verbindung nach Anspruch 1, bei der es sich um 6,9-Bis[(2-aminoethyl)amino]benzo[g]isochinolin-5,10-dion oder ein Salz desselben handelt.

**5.** Verbindung nach Anspruch 1, wobei das Salz ein Dimaleat-Salz ist.

**6.** Pharmazeutische Zusammensetzung, umfassend eine Verbindung nach den Ansprüchen 1 bis 5 und ein pharmazeutisch annehmbares Verdünnungsmittel oder einen solchen Träger.

**7.** Pharmazeutische Zusammensetzung, umfassend 6,9-Bis[(2-aminoethyl)amino]benzo[g]isochinolin-5,10-dion oder ein Salz desselben und ein pharmazeutisch annehmbares Verdünnungsmittel oder einen solchen Träger.

**8.** Pharmazeutische Zusammensetzung nach Anspruch 7, wobei das Salz ein Dimaleat-Salz ist.

**9.** Verwendung einer Verbindung nach den Ansprüchen 1 bis 5 zur Herstellung eines Medikaments für die Behandlung von Tumoren bei Säugern, die einer solchen Behandlung bedürfen.

**10.** Verfahren zur Synthese einer Verbindung gemäß Formel I

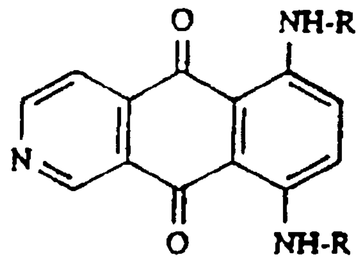

(I),

worin R

(a) $-(CH_2)_p-NH_2$ ist, und p 2, 3 oder 4 ist, oder
(b) $-(CH_2)_p-NR_2R_3$ ist, und p 2, 3 oder 4 ist, und worin $R_2$ und $R_3$ ein $C_1$-$C_6$-Alkyl sind oder zusammen mit dem Stickstoff-Atom einen heterocyclischen Ring bilden, ausgewählt aus der Gruppe bestehend aus 1-Ethylenimin, 1-Pyrrolidin, 4-Morpholin, 1-Piperazin, 4-Methyl-1-piperazin, 4-Benzyl-1-piperazin und 1-Piperidin, oder
(c) $-(CH_2)_p-NR_2R_3$ ist, und p 2, 3 oder 4 ist, und worin $R_2$ Wasserstoff ist und $R_3$ $C_1$-$C_6$-Alkyl ist, oder
(d)

$$-(CH_2)_p-NH-\overset{\overset{\displaystyle NH}{\|}}{C}-NH_2$$

ist, und p 2, 3 oder 4 ist, oder
(e) $-(CH_2)_p-NH-(CH_2)_q-OH$ ist, und p und q unabhängig voneinander 2, 3 oder 4 sind, oder
(f) $-(CH_2)_p-OH$ ist, und p 2, 3 oder 4 ist, oder
(g) $-(CH_2)_p-O-(CH_2)_q-OH$ ist, und p und q unabhängig voneinander 2, 3 oder 4 sind,

als freie Base und deren Salze mit pharmazeutisch annehmbaren Säuren, umfassend die Schritte

a) Umsetzen von Pyridin-3,4-dicarbonsäureanhydrid mit 1,4-Difluorbenzol;
b) Cyclisieren des Produkts von Schritt a) in rauchender Schwefelsäure bei höherer Temperatur; und
c) Substitution von Fluorid durch Zugabe eines Amins der Formel $R'-NH_2$, worin R' das gleiche wie das durch R definierte ist.

**11.** Verfahren nach Anspruch 10, wobei eine in Schritt c) erhaltene Verbindung die Formel (Ia)

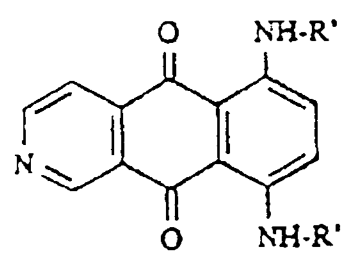

(Ia)

aufweist, und wobei R' eine andere als die durch R definierte Bedeutung hat, und des weiteren den Schritt der Überführung von R' in R umfaßt.

**12.** Verfahren nach Anspruch 10, des weiteren umfassend den Schritt der Überführung von R' in der in Schritt c) erhaltenen Verbindung in R, wodurch eine andere Verbindung definiert wird.

**13.** Verfahren nach Anspruch 10, des weiteren umfassend den Schritt, daß die in Schritt c) erhaltene Verbindung einer Salzbildung und/oder Solvatation oder Trennung der Isomere derselben unterzogen wird.

**14.** Verfahren zur Synthese einer Verbindung gemäß Formel I von Anspruch 10, wobei R

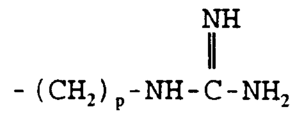

ist und p 2, 3 oder 4 ist, durch Umsetzen einer Verbindung der Formel L, worin R $-(CH_2)_p-NH_2$ ist und p 2, 3 oder 4 ist, mit einer Verbindung der Formel $H_2N-C(=NH)SO_3H$.

**15.** Verfahren zur Synthese der Verbindung von Anspruch 3 durch Umsetzen des nach dem Verfahren von Anspruch 10 erhaltenen 6,9-Bis[(2-aminoethyl)amino]benzo[g]isochinolin-5,10-dions mit Acetanhydrid.

**Revendications**

**1.** Composé selon la formule I:

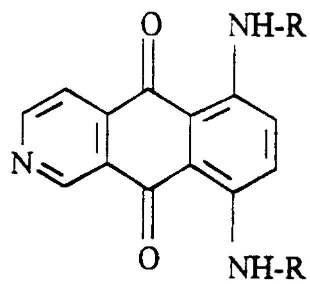

(I),

dans laquelle R est

(a) $-(CH_2)_p-NH_2$ et p vaut 2, 3 ou 4, ou
(b) $-(CH_2)_p-NR_2R_3$ et p vaut 2, 3 ou 4 et dans laquelle $R_2$ et $R_3$ sont un groupe alkyle en $C_1$ à $C_6$ ou pris ensemble avec un atome d'azote forment un cycle hétérocyclique choisi dans le groupe formé par la 1-éthylèneimine, la 1-pyrrolidine, la 4-morpholine, la 1-pipérazine, la 4-méthyl-1-pipérazine, la 4-benzyl-1-pipérazine et la 1-pipéridine, ou
(c) $-(CH_2)_pNR_2R_3$ et p vaut 2, 3 ou 4 et dans laquelle $R_2$ est un atome d'hydrogène et $R_3$ est un groupe alkyle en $C_1$ à $C_6$, ou
(d)

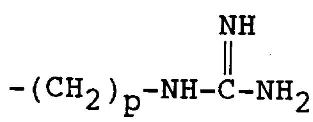

et p vaut 2, 3 ou 4, ou
(e) $-(CH_2)_p-NH-(CH_2)_q-OH$ et p et q valent indépendamment 2, 3 ou 4, ou
(f) $-(CH_2)_p-OH$ et p vaut 2, 3 ou 4, ou
(g) $-(CH_2)_p-O-(CH_2)_q-OH$ et p et q valent indépendamment 2, 3 ou 4

sous forme de bases libres et leurs sels avec des acides acceptables sur le plan pharmaceutique.

**2.** Composé selon la revendication 1, choisi dans le groupe formé par:

- la 6,9-bis([2-(amino)éthyl]amino)benzo[g]isoquinoléine-5,10-dione;
- la 6,9-bis([2-(4'-morpholino)éthyl]amino)benzo[g]isoquinoléine-5,10-dione;
- la 6,9-bis([2-(diméthylamino)éthyl]amino)benzo[g]isoquinoléine-5,10-dione;
- la 6,9-bis([2-(diéthylamino)éthyl]amino)benzo[g]isoquinoléine-5,10-dione;
- la 6,9-bis([2-[di(sec-propyl)amino]éthyl]amino)benzo[g]isoquinoléine-5,10-dione;
- la 6,9-bis([2-(1'-pyrrolidino)éthyl[amino)benzo[g]isoquinoléine-5,10-dione;
- la 6,9-bis([2-(1'-aziridino)éthyl]amino)benzo[g]isoquinoléine-5,10-dione;

- la 6,9-bis([2-méthanesulfonyloxy)éthyl]amino)benzo[g]isoquinoléine-5,10-dione;
- la 6,9-bis([4-(amino)butyl]amino)benzo[g]isoquinoléine-5,10-dione;
- la 6,9-bis([3-(amino)propyl]amino)benzo[g]isoquinoléine-5,10-dione;
- la 6,9-bis([2-[(2-hydroxyéthyl)amino]éthyl]amino)benzo[g]isoquinoléine-5,10-dione;
- la 6,9-bis([3-(diméthylamino)propyl]amino)benzo[g]isoquinoléine-5,10-dione;
- la 6,9-bis([(2-hydroxy)éthyl]amino)benzo[g]isoquinoléine-5,10-dione;
- la 6,9-bis([(2-(2-hydroxyéthoxy)éthyl]amino)benzo[g]isoquinoléine-5,10-dione;
- la 6,9-bis([2-(méthylamino)éthyl]amino)benzo[g]isoquinoléine-5,10-dione;
- la 6,9-bis([2-(éthylamino)éthyl]amino)benzo[g]isoquinoléine-5,10-dione;
- la 6,9-bis([2-(n-propylamino)éthyl]amino)benzo[g]isoquinoléine-5,10-dione;
- la 6,9-bis([2-(sec-propylamino)éthyl]amino)benzo[g]isoquinoléine-5,10-dione;
- la 6,9-bis([2-(guanidino)éthyl]amino)benzo[g]isoquinoléine-5,10-dione;
- la 6,9-bis([(2-amino-2,2-diméthyl)éthyl]amino)benzo[g]isoquinoléine-5,10-dione.

**3.** 6,9-bis([2-(acétylamino)éthyl]amino)benzo[g]isoquinoléine-5,10-dione sous forme de base libre et ses sels avec des acides acceptables sur le plan pharmaceutique.

**4.** Composé selon la revendication 1 qui est la 6,9-bis([2-(amino)éthyl]amino)benzo[g]isoquinoléine-5,10-dione ou un de ses sels.

**5.** Composé selon la revendication 1, dans lequel ledit sel est un dimaléate.

**6.** Composition pharmaceutique comprenant un composé selon les revendications 1 à 5 et un diluant ou excipient acceptable sur le plan pharmaceutique.

**7.** Composition pharmaceutique comprenant la 6,9-bis([2-(amino)éthyl]amino]amino)benzo[g]isoquinoléine-5,10-dione ou un de ses sels et un diluant ou un excipient acceptable sur le plan pharmaceutique.

**8.** Composition pharmaceutique selon la revendication 7, dans laquelle ledit sel et un dimaléate.

**9.** Utilisation d'un composé selon les revendications 1 à 5 pour la production d'un médicament destiné au traitement de tumeurs chez un mammifère nécessitant un tel traitement.

**10.** Procédé pour la synthèse d'un composé selon la formule I:

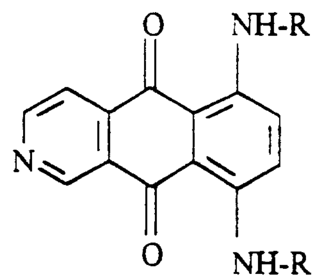

(I),

dans laquelle R est

(a) $-(CH_2)_p-NH_2$ et p vaut 2, 3 ou 4, ou
(b) $-(CH_2)_p-NR_2R_3$ et p vaut 2, 3 ou 4 et dans laquelle $R_2$ et $R_3$ sont un groupe alkyle en $C_1$ à $C_6$ ou pris ensemble avec un atome d'azote forment un cycle hétérocyclique choisi dans le groupe formé par la 1-éthylèneimine, la 1-pyrrolidine, la 4-morpholinè, la 1-pipérazine, la 4-méthyl-1-pipérazine, la 4-benzyl-1-pipérazine et la 1-pipéridine, ou
(c) $-(CH_2)_p-NR_2R_3$ et p vaut 2, 3 ou 4 et dans laquelle $R_2$ est un atome d'hydrogène et $R_3$ est un groupe alkyle en $C_1$ à $C_6$, ou
(d)

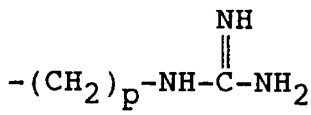

et p vaut 2, 3 ou 4, ou

(e) $-(CH_2)_p-NH-(CH_2)_q-OH$ et p et q valent indépendamment 2, 3 ou 4, ou
(f) $-(CH_2)_p-OH$ et p vaut 2, 3 ou 4, ou
(g) $-(CH_2)_p-O-(CH_2)_q-OH$ et p et q valent indépendamment 2, 3 ou 4

sous forme de bases libres et leurs sels avec des acides acceptables sur le plan pharmaceutique, comprenant les étapes consistant:

a) à faire réagir l'anhydride pyridine-3,4-dicarboxylique avec le 1,4-difluorobenzène;
b) à cycliser le produit de l'étape a) dans l'acide sulfurique fumant à température élevée; et
c) à déplacer le fluorure par addition d'une amine de formule $R'-NH_2$, dans laquelle R' est le même que celui défini par R.

**11.** Procédé selon la revendication 10, dans lequel un composé obtenu dans l'étape (c) possède la formule (Ia):

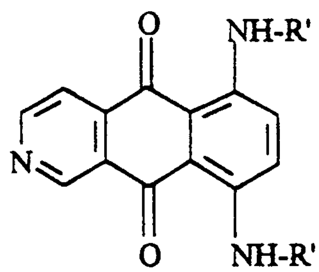

(Ia),

et dans laquelle R' a une signification autre que celle définie par R et comprenant de plus l'étape consistant à convertir R' en R.

**12.** Procédé selon la revendication 10, comprenant de plus l'étape consistant à convertir R' dans le composé obtenu dans l'étape (c) en un autre composé défini par R.

**13.** Procédé selon la revendication 10, comprenant de plus l'étape consistant à soumettre le composé obtenu dans l'étape (c) à une salification et/ou à une solvatation ou à une séparation de ses isomères.

**14.** Procédé pour la synthèse d'un composé selon la formule I de la revendication 10, dans lequel R est

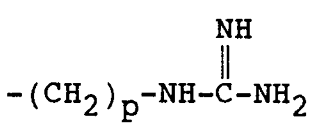

et p vaut 2, 3 ou 4 en faisant réagir un composé de formule I dans laquelle R est $-(CH_2)_p-NH_2-$ et p vaut 2, 3 ou 4 avec un composé ayant la formule $H_2N-C(=NH)SO_3H$.

**15.** Procédé pour la synthèse du composé selon la revendication 3 consistant a faire réagir la 6,9-bis([2-(amino)éthyl)amino]benzo[g]isoquinoléine-5,10-dione obtenue selon le procédé de la revendication 10 avec l'anhydride acétique.